# EUROPEAN PATENT APPLICATION

(11) **EP 0 881 220 A1**
(43) Date of publication of application: **02.12.1998**
(21) Application number: 97902691.1
(22) Date of filing: 14.02.1997
(51) Int. Cl.: C07D 285/36, C07D 291/08, C07D 513/04, C07D 513/12, C07D 515/04, C07D 515/14, A61K 31/55

(54) **DIARYLSULTAM DERIVATIVES**

(30) Priority: 15.02.1996 JP 27745/96
(71) Applicant: MITSUBISHI CHEMICAL CORPORATION, Chiyoda-ku, Tokyo 100-0005 (JP)
(72) Inventor: ROCHER, Jean-Philippe Mitsubishi Chemical Corp., Yokohama-shi Kanagawa 227 227 (JP)
(74) Representative: Godemeyer, Thomas, Dr.
(86) International application number: JP9700400
(87) International publication number: WO9730038

(57) **Abstract**

Compounds represented by the formula: A-C(X)(Y)-C(R₁)(R₂)-Z, wherein Z represents, for example, a group of -N(R₃)-(CH₂)ₚ-B wherein R₃ is alkyl group or other; p is integer of 3 to 8; B is a group represented by the following formula: 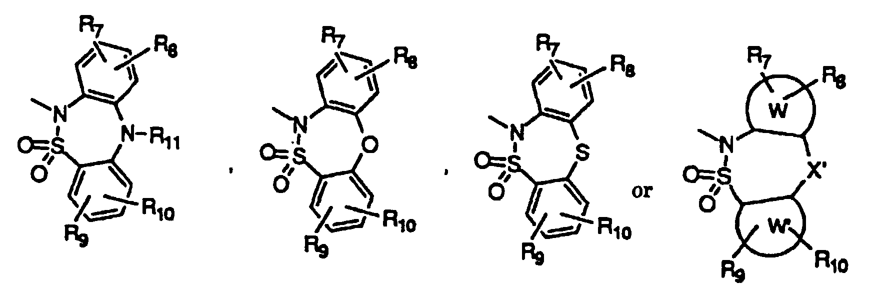 wherein R₇, R₈, R₉ and R₁₀ represent hydrogen atom, halogen atom, alkyl group or other; X' is -S-, S(O)-, -S(O)₂-, -O-, or -N(R₁₁)- wherein R₁₁ is hydrogen atom, alkyl group or acyl group; W and W' represent benzene ring, or 5- to 7-membered heteroaryl group; X is cycloalkyl group, aryl group or other; Y is hydrogen atom, alkyl group, alkenyl group or other; A is -O-R₆ wherein R₆ is hydrogen atom, alkyl group, cycloalkyl group etc.; and R₁ and R₂ represent hydrogen atom, alkyl group or other.

The compounds have high affinity and selectivity for sigma 2 binding site, and therefore they are useful as selective sigma 2 ligands for therapeutic and/or preventive treatment of various diseases and symptoms caused by sigma 2 ligands.

## Description

### Technical Field

The present invention relates to novel diarylsultam derivatives. More precisely, the present invention relates to novel compounds which can act as selective sigma 2 ligands, and pharmaceutical compositions comprising said compound as an active ingredient and useful for treatment of central nervous system disorders and several other disorders.

### Background Art

The recently identified brain sigma receptors/binding sites are potential targets for development of antipsychotic drugs that lack the adverse effects associated with currently available antipsychotic drugs having antagonistic effect on dopamine D2 receptor [J. M. Walker, W. D. Bowen, F. O. Walker, R. R. Matsumoto, B. de Costa and K. C. Rice, Pharmacological Reviews, 1990, 42, 355-402; G. Debonnel, J. Psychiatr. Neurosci., 1993, 18, 4, 157-172]. The term "receptor" as used herein refers to membrane-bound receptors and to other binding sites. The existence of at least two sigma receptor subtypes, sigma 1 and sigma 2, has been confirmed and a classification of sigma binding sites has been proposed [R. Quirion, W. D. Bowen, Y. Itzhak, J. L. Junien, J. M. Musacchio, R. B. Rothman, T. P. Su, W. Tam and D. P. Taylor, TiPS, 1992, 13, 85-86].

Sigma 1 binding sites are characterized by high affinity for haloperidol, di-o-tolylguanidine (DTG) and (+)-benzomorphans such as (+)-pentazocine. Sigma 2 binding sites are characterized by high affinity for haloperidol and DTG but low affinity for the (+)-benzomorphans. Sigma 1 ligands have actions on gastrointestinal tract; moreover, sigma 1 sites appear to mediate the inhibition by sigma ligands of the muscarinic acetylcholine receptor phosphoinositide response. The sigma 1 binding sites are not only present in the brain, but also on splenocytes. Such sigma ligands may inadvertently supress the immune system [H. H. Garza, S. Mayo, W. D. Bowen, B. R. DeCosta and D. J. J. Carr, J. of Immunology, 1993, 151, 9, 4672-4680]. Sigma 2 binding sites are abundant in liver [A. E. Bruce, S. B. Hellewell and W. D. Bowen, Soc. Neurosci. Abstr., 1990, 16, 370; A. S. Basile, I. A. Paul and B. DeCosta, Eur. J. Pharmacol. Mol. Pharm. Sect., 1992, 227, 95-98], kidney [W. D. Bowen, G. Feinstein and J. S. Orringer, Soc. Neurosci. Abstr., 1992, 18, 456, abstract 195.8], and heart [M. Dumont and S. Lemaire, Eur. J. Pharmacol., 1991, 209, 245-248].

In the brain, sigma 2 binding sites are present in hypothalamus, cerebellum, pith bridge, and pons medulla. In rat brain, they are more abundant than sigma 1 sites in hippocampus, frontal and posterior cortex [D. J. Mc Cann, A. D. Weissmann and T. P. Su, Soc. Neurosci. abstr., 1992, 18, 22, abstract 16.5]. Guinea pig hippocampus synaptosome posesses also sigma 2 binding sites which are selectively labeled by [³H] BIMU [D. W. Bonhaus, D. N. Loury, L. B. Jakeman, Z. To, A. DeSouza, R. M. Eglen and E. H. F. Wong, J. Pharmacol. Exp. Ther., 1993, 267, 2, 961-970]. The association of sigma 2 binding sites with cortical and limbic areas provide support to the interest of such compounds in the treatment of psychiatric disorders [D. C. Mash and C. P. Zabetian, Synapse, 1992, 12, 195-205]. Sigma 2 binding sites have been suspected to mediate motor effects especially dystonia [R. R. Matsumoto, M. K. Hemstreet, N. L. Lai, A. Thurkauf, B. R. DeCosta, K. C. Rice, S. B. Hellewell, W. D. Bowen and J. M. Walker, Pharmacol. Biochem. Behav., 1990, 36, 151-155]. However, there is no evidence for such effect in primate models of extrapyramidal dysfunction [L. T. Meltzer, C. L. Christoffersen, K. A. Serpa, T. A. Pugsley, A. Razmpour and T. G. Heffner, Neuropharmacology, 1992, 31, 9, 961-967].

The clinically effective dopaminergic antipsychotic haloperidol shows a high affinity to the two sigma subtypes; however, the CNS active reduced metabolite of haloperidol shows a better affinity and selectivity for sigma 2 receptors towards dopaminergic D2 receptors as compared to haloperidol [J. C. Jaen, B. W. Caprathe, T. A. Pugsley, L. D. Wise and H. Akunne, J. Med. Chem., 1993, 36, 3929-3936]. In fact, the understanding of the pharmacological significance, distribution and function of sigma 2 binding sites has been hampered by the lack of selective agents. In addition, a recent study demonstrates the role of the sigma 2 site in the mediation of ileal function [G. G. Kinney, E.W. Haris, R. Ray and T.J. Hudzik, Europ. J. Pharmacol., 1995, 294, 547-553]. These data suggest tha therapeutic interest of selective sigma 2 ligands for the treatment of irritable bowel syndrome.

Few compounds bind with a high affinity to sigma 2 binding sites[M. Abou-Gharbia, S. Y. Ablordeppey and R. A. Glennon, Annual Reports in Medicinal Chemistry 1993, 28, 1-10]. For example, ifenprodil has a marked selectivity for sigma 2 sites but it binds also to polyamine and alpha-1 receptors [K. Hashimoto, C. R. Mantione, M. R. Spada, J. L. Neumayer and E. D. London, Eur. J. Pharmacol. Mol. Pharm. Sect., 1994, 266, 66-77]. *Threo*-ifenprodil was slightly more selective than *erythro*-ifenprodil for sigma 2 sites [K. Hashimoto and E. D. London, Eur. J. Pharm., 1995, 273, 307-310].

[³H] BIMU has been proposed as a selective sigma 2 ligand with Ki=32 nM [D. W. Bonhaus, D. N. Loury, L. B. Jakeman, Z. To, A. DeSouza, R. M. Eglen and E. H. F. Wong, J. Pharmacol. Exp. Ther., 1993, 267, 2, 961-970]; however, despite a good selectivity towards sigma 2, this compound is also a highly potent 5-HT3 and 5-HT4 serotonin ligand. WO93/00313 relates to sigma receptor ligands and their sigma 1/sigma 2 selectivity; three compounds present an interesting sigma 2 selectivity, they belong to the phenyl piperazine class and are also high affinity 5-HT1A serotonin ligands [R.A. Glennon, N. A. Naiman, R. A. Lyon and M. Titeler, J. Med. Chem., 1988, 31, 1968-1971].

Sigma binding site ligands having affinity (Ki) below 1 nM have been presented as sigma 2 preferential ligands [J. Perregaard, E. K. Moltzen, E. Meier and C. Sanchez, J. Med. Chem., 1995, 38, 1998-2008; E. K. Moltzen, J. Perregaard and E. Meier, J. Med. Chem., 1995, 38, 2009-2017]; but some compounds present a moderate affinity for certain monoaminergic receptors. The 5,6,7,8,9,10-hexahydro-7,10-iminocyclohept[b]indoles have been identified as selective sigma 2 ligands with moderate to high affinity [R. E. Mewshaw, R. G. Sherrill, R. M. Mathew, C. Kaiser, M. A. Bailey and E. W. Karbon, J. Med. Chem., 1993, 36, 343-352].

As a new approach to sigma 2 ligands has been disclosed: polyamino sigma ligands described were moderately potent and selective for sigma 2 sites [B. R. DeCosta, X. S. He, C. Dominguez, J. Cutts, W. Williams and W. D. Bowen, J. Med. Chem., 1994, 37, 314-321]. Recently, (E)-8-benzylidene-5-phenylmorphans have been reported as a new class of sigma 2 selective ligands [W. D. Bowen, C. M. Bertha, B. J. Vilner and K. C. Rice, Eur. J. Pharm., 1995, 278, 257-260; C. M. Bertha, B. J. Vilner, M. V. Mattson, W. D. Bowen, K. Becketts, H. Xu, R. B. Rothman, J. L. Flippen-Anderson and K.C. Rice, J. Med. Chem., 1995, 38, 4776-4785]; however, the compounds shared a high affinity for the µ-opioid receptor.

The indole alkaloid Ibogain has been shown to be a moderate sigma 2 selective ligand [W. D. Bowen, B. J. Vilner, W. Williams, C. M. Bertha, M. E. Kuehne and A. E. Jacobson, Eur. J. Pharm. 1995, 279, R1-R3]. This natural product presents "endabuse" properties; futhermore, the moderate affinity sigma 2 preferential ligands BMY14802 and rimcazole can block the stimulant effects of cocaine [M. Menkel, P. Terry, M. Pontecorvo, J. L. Katz and J. M. Witkin, Eur. J. Pharm., 1991, 201, 251-252]. These studies deserves the therapeutic potential of sigma 2 ligands for the treatment of drug abuse.

Extensive literature exists relating to heteroaryl alkylamino derivatives; however, there is very little data concerning diaryl seven-membered sultam series. Dibenzothiadiazepine derivatives are described by Gianotti et al. [D. Gianotti, G. Viti, P. Sbraci, V. Pestellini, G. Volterra, F. Borsini, A. Lecci, A. Meli, P. Dapporto and P. Paoli, J. Med. Chem., 1991, 34, 1356-1362]. The synthesis of dibenzoxathiazepine ring was conducted according to different routes of synthesis [R. A. Abramovitch, C. I. Azogu and I. T. McMaster, J. Am. Chem. Soc. 1969, 91, 5, 1219-1220; R. A. Abramovitch, C. I. Azogu, I. T. McMaster and D. P. Vanderpool, J. Org. Chem., 1978, 43, 6, 1218-1226; P. S. Dewar, A. R. Forrester and R. H. Thomson, J. Chem. Soc. Perkin I, 1972, 2862-2865; Belg. patent 622 185]. Japanese Patent Publication No.(Sho)46-3060/1971 describes some alkylamino derivatives of diaryl ether sultams. EP 546388 discloses heteroaryl methylpiperidino derivatives of chromans without examples of such sultam compounds. WO93/22309 presents 4-imidomethyl-1-[2'-phenyl-2'-oxoethyl]-piperidines as serotonin 5-HT2 antagonists.

Numerous phenylethanolamino derivatives exhibit important pharmaceutical properties [Pharmaceutical Chemistry Vol. 1: Drug Synthesis, H. J. Roth, A. Kleemann and T. Beisswenger, Ellis Horwood Limited, Chichester England, 37-69]. For example, benzylpiperidino derivatives like ifenprodil and its derivatives belong to this class and are known to interact with sigma, adrenergic and glutamatergic receptors [B. L. Chenard, I. A. Shalaby, B. K. Koe, R. T. Ronau, T. W. Butler, M. A. Prochniak, A. W. Schmidt and C. B. Fox, J. Med. Chem., 1991, 34, 3085-3090]. However, the compounds described above do not show the sigma 2 binding site selectivity and affinity.

### Disclosure of the Invention

An object of the present invention is to provide novel compounds having excellent selectivity and affinity for the sigma 2 binding site. Another object of the present invention is to provide medicaments useful for the treatment and/or prevention of diseases affected or facilitated by the neuromodulatory effect of sigma 2 ligands.

The present invention recides in the discovery of a group of selective and high affinity sigma 2 binding site ligands, i.e., the present invention relates to substituted (preferentially adamantyl, cycloalkyl or aryl)hydroxyethylamino derivatives. The inhibition constant Ki for sigma 2 binding site is 50 nM or lower, and these new ligands have at least 5 fold greater affinity for sigma 2 than sigma 1 binding sites and dopaminergic D2 receptors. Surprisingly, the ethanolamino tricyclic sultam derivatives of the present invention show completely different and selective binding profile from those of the prior disclosed phenyl ethanolamines, which makes the compounds of the present invention advantageous over compounds of the prior art.

The present invention thus provides diarylsultam derivatives represented by the following formula: A-C(X)(Y)-C(R₁)(R₂)-Z and their salts, and hydrates thereof and solvates thereof. In the formula, Z represents a group represented by formula: 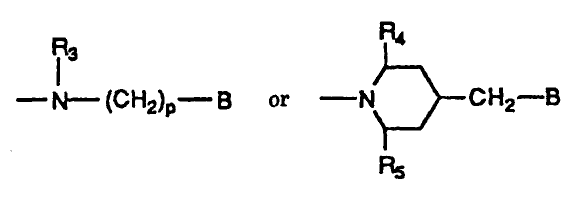 wherein:
R₃ represents an alkyl group, a cycloalkyl group, a hydroxyalkyl group, or an alkenyl group;
p represents an integer of from 3 to 8;
R₄ and R₅ independently represent hydrogen atom or an alkyl group, or R₄ and R₅ together with the intervening atom represent a 5- to 7-membered heterocyclic ring; and
B represents a heteroaryl group of formula: 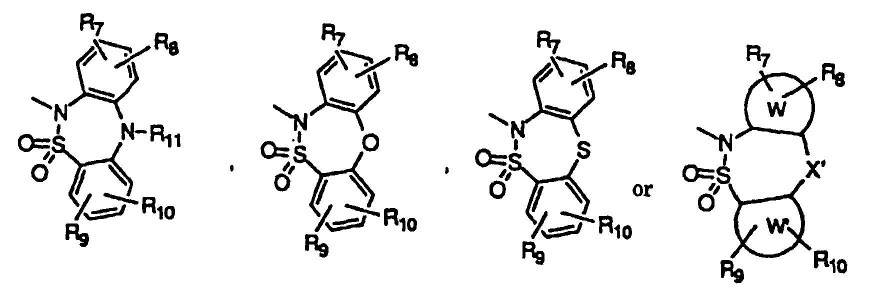 wherein:
R₇, R₈, R₉ and R₁₀ independently represent a substituent selected from the group consisting of hydrogen atom, a halogen atom, nitro group, a cycloalkyl group, an alkenyl group, an alkyl group optionally substituted with 1 to 3 fluorine atoms, hydroxyl group, an alkoxy group optionally substituted with 1 to 3 fluorine atoms, a phenyl group, amino group, an alkylamino group, carbamoyl group, sulfamoyl group, an acyl group, cyano group and an alkynyl group;
X' represents -S-, S(O)-, -S(O)₂-, -O-, or -N(R₁₁)- wherein R₁₁ represents hydrogen atom, an alkyl group or an acyl group; and
W and W' independently represent benzene ring or a 5- to 7-membered heteroaryl group containing one oxygen atom, one sulfur atom, or one or two nitrogen atoms, provided that at least one of W and W' represents the aforementioned heteroaryl group;
X represents a cycloalkyl group, a cycloalkyl-substituted alkyl group, an alkyl-substituted cycloalkyl group, a polycyclic cycloalkyl group, an aryl group, or a heteroaryl group, wherein the aryl group and the heteroaryl group may be substituted with one or more substituents independently selected from the group consisting of a halogen atom, nitro group, a cycloalkyl group, an alkenyl group, an alkyl group optionally substituted with 1 to 3 fluorine atoms, hydroxyl group, an alkoxy group optionally substituted with 1 to 3 fluorine atoms, phenyl group, amino group, an alkylamino group, carboxyl group, carbamoyl group, sulfamoyl group, an acyl group, cyano group, and an alkynyl group;
Y represents hydrogen atom, an alkyl group, an alkenyl group, an alkynyl group, a cycloalkyl group, a cycloalkyl-substituted alkyl group, adamantyl group, an aryl group, an aryl-substituted alkyl group, a heteroaryl group or a heteroaryl-substituted alkyl group, wherein the aryl group and the heteroaryl group may be substituted with one or more substituents independently selected from the group consisting of a halogen atom, nitro group, a cycloalkyl group, an alkenyl group, an alkyl group optionally substituted with 1 to 3 fluorine atoms, hydroxyl group, an alkoxy group optionally substituted with 1 to 3 fluorine atoms, phenyl group, amino group, an alkylamino group, carbamoyl group, sulfamoyl group, an acyl group, cyano group and an alkynyl group;
A is a group represented by -O-R₆ wherein R₆ represents hydrogen atom, an alkyl group, a cycloalkyl group, a cycloalkyl-substituted alkyl group, an alkenyl group, an aryl group, an aryl-substituted alkyl group, a hydroxyalkyl group, an acyl group, or benzoyl group;
Y and A may form oxo group or hydroxylimino group; and
R₁ and R₂, which may be the same or different, independently represent hydrogen atom, an alkyl group, a cycloalkyl group, a hydroxyalkyl group or an alkenyl group.

According to other aspect of the present invention, there are provided medicaments comprising the aforementioned diarylsultam derivatives or salts thereof, or hydrates thereof or solvates thereof; and pharmaceutical compositions comprising the aforementioned diarylsultam derivatives or salts thereof, or hydrates thereof or solvates thereof as an active ingredient. The medicaments and pharmaceutical compositions are useful for the therapeutic and/or preventive treatment of diseases and/or symptoms of mammals, including human, which are affected or facilitated by the neuromodulatory effect of sigma 2 ligands. For example, they are useful as therapeutic and preventive medicaments for central nervous system diseases, gastrointestinal diseases, cardiovascular system diseases and other. As another embodiment of the present invention, there is also provided selective sigma 2 ligands consisting of the aforementioned diarylsultam derivatives or salts thereof, or hydrates thereof or solvates thereof.

According to further aspects of the present invention, there are provided use of a substance selected from the aforementioned diarylsultam derivatives and salts thereof, and hydrates thereof and solvates thereof for the manufacture of the aforementioned pharmaceutical composition; and methods for therapeutic and/or preventive treatment of diseases and/or symptoms affected or facilitated by neutomodulatory effect of sigma 2 ligands, which comprise the step of administering to a patient a therapeutically and/or preventively effective amount of a substance selected from the aforementioned diarylsultam derivatives and salts thereof, and hydrates thereof and solvates thereof.

### Best Mode for Carrying Out the Invention

In the above definition, examples of the alkyl group include, for example, alkyl groups having 1 to 6 carbon atoms such as methyl group, ethyl group, n-propyl group, isopropyl group, n-butyl group, isobutyl group, sec-butyl group, tert-butyl group, n-pentyl group, isopentyl group, neopentyl group, tert-pentyl group and n-hexyl group. Examples of the cycloalkyl group include, for example, cycloalkyl groups having 3 to 6 carbon atoms such as cyclopropyl group, cyclobutyl group, cyclopentyl group and cyclohexyl group. Examples of the cycloalkyl-substituted alkyl group include, for example, alkyl groups having 1 to 3 carbon atoms substituted with a cycloalkyl group having 3 to 6 carbon atoms such as cyclopropylmethyl group, cyclopropylethyl, cyclopropylpropyl group, cyclobutylmethyl group, cyclobutylethyl, cyclopentylmethyl group and cyclohexylmethyl group.

Examples of the polycyclic cycloalkyl group include, for example, polycycloalkyl groups having 5 to 12 carbon atoms such as norbornyl group and adamantyl group. Examples of the alkenyl group include, for example, those having 3 to 6 carbon atoms such as 1-propenyl group, allyl group, isopropenyl group, 1-butenyl group, 3-butenyl group, 1-pentenyl group, 2-pentenyl group, 3-pentenyl group, 4-pentenyl group, 1-hexenyl group and 2-hexenyl group. Examples of the alkynyl group include for example, those having 3 to 6 carbon atoms such as 2-propynyl group, 3-butynyl group, 4-pentynyl group and 5-hexynyl group. Examples of the alkoxy group include, for example, those having 1 to 6 carbon atoms such as methoxy group, ethoxy group, n-propoxy group, isopropoxy group, n-butoxy group, isobutoxy group, tert-butoxy group, n-pentyloxy group and n-hexyloxy group. The halogen atom may be any of fluorine atom, chlorine atom, bromine atom and iodine atom.

Examples of the alkylamino group include, for example, alkylamino groups having 1 to 6 carbon atoms such as methylamino group, ethylamino group, propylamino group, butylamino group, pentylamino group, hexylamino group, dimethylamino group, methylethylamino group, diethylamino group and dipropyl amino group. Examples of the acyl group include, for example, alkylcarboxyl groups having 2 to 6 carbon atoms such as acetyl group, propionyl group and butyryl group. Examples of the hydroxyalkyl group include, for example, hydroxyalkyl groups having 2 to 6 carbon atoms such as 2-hydroxyethyl group, 3-hydroxypropyl group, 4-hydroxybutyl group, 5-hydroxypentyl group and 6-hydroxyhexyl group.

Examples of the aryl group include, for example, those aryl groups having 6-10 carbon atoms as ring-membered atoms such as phenyl group, 1-naphthyl group and 2-naphthyl group. Examples of the arylalkyl group include, for example, alkyl groups having 1 to 3 carbon atoms substituted with an aryl group having 6-10 carbon atoms such as benzyl group, 1-phenylethyl group, 2-phenylethyl group, 1-phenylpropyl group, 2-phenylpropyl group, 3-phenylpropyl group, 1-naphthylmethyl group and 2-naphthylmethyl group.

As the heteroaryl group, 5- to 10-membered heterocyclic groups containing 1 to 4 heteroatoms as ring-membered atom(s) selected from oxygen atom, sulfur atom and nitrogen atom. Examples include furyl group (furan ring), benzofuranyl group (benzofuran ring), isobenzofuranyl group (isobenzofuran ring), thienyl group (thiophene ring), benzothiophenyl group (benzothiophene ring), pyrrolyl group (pyrrole ring), imidazolyl group (imidazole ring), pyrazolyl group (pyrazole ring), thiazolyl group (thiazole ring), benzothiazolyl group (benzothiazole ring), isothiazolyl group (isothiazole ring), benzoisothiazolyl group (benzoisothiazole ring), triazolyl group (triazole ring), tetrazolyl group (tetrazole ring), pyridyl group (pyridine ring), pyrazinyl group (pyrazine ring), pyrimidinyl group (pyrimidine ring), pyridazinyl group (pyridazine ring), indolyl group (indole ring), isoindolyl group (isoindole ring), benzimidazolyl group (benzimidazole ring), purinyl group (purine ring), quinolyl group (quinoline ring), phthalazinyl group (phthalazine ring), naphtylidinyl group (naphtylidine ring), quinoxalinyl group (quinoxaline ring), cinnolinyl group (cinnoline ring), pteridinyl group (pteridine ring), oxazolyl group (oxazole ring), isoxazolyl group (isoxazole ring), benzoxazolyl group (benzoxazole ring), benzoisoxazolyl group (benzoisoxazole ring), furazanyl group (furazane ring) and the like.

Examples of the heteroarylalkyl group include, for example, alkyl groups having 1 to 3 carbon atoms substituted with a heteroaryl group selected from those mentioned above such as 2-furylmethyl group, 3-furylmethyl group, 2-thienylmethyl group, 3-thienylmethyl group, 1-imidazolylmethyl group, 2-imidazolylmethyl group, 2-thiazolylmethyl group, 1-pyridylmethyl group, 2-pyridylmethyl group, 3-pyridylmethyl group, 4-pyridylmethyl group, 1-quinolylmethyl group, and 2-quinolylmethyl group.

Among the compounds of formula (I) defined above, preferred compounds of the present invention include those wherein:
R₃ is an alkyl group having 1 to 3 carbon atoms, a cycloalkyl group having 3 to 6 carbon atoms, a hydroxyalkyl group having 2 or 3 carbon atoms or an alkenyl group having 3 to 6 carbon atoms;
R₇, R₈, R₉ and R₁₀ are independently selected from the group consisting of hydrogen atom, a halogen atom, nitro group, an alkyl group having 1 to 6 carbon atoms optionally substituted with 1 to 3 fluorine atoms, hydroxyl group, an alkoxy group having 1 to 6 carbon atoms optionally substituted with 1 to 3 fluorine atoms, phenyl group, amino group, an alkylamino group having 1 to 6 carbon atoms, carbamoyl group, sulfamoyl group, an acyl group having 2 to 6 carbon atoms and cyano group;
R₁₁ is selected from hydrogen atom, an alkyl group having 1 to 6 carbon atoms and an acyl group having 2 to 6 carbon atoms;
X represents a cycloalkyl group having 3 to 6 carbon atoms, an alkyl group having 1 to 3 carbon atoms substituted with a cycloalkyl group having 3 to 6 carbon atoms, a cycloalkyl group having 3 to 6 carbon atoms substituted with an alkyl group having 1 to 3 carbon atoms, a polycycloalkyl group having 5 to 12 carbon atoms, an aryl group or a heteroaryl group, wherein the aryl group and the heteroaryl group may be substituted with 1 to 3 substituents independently selected from the group consisting of a halogen atom, nitro group, a cycloalkyl group having 3 to 6 carbon atoms, an alkenyl group having 3 to 6 carbon atoms, an alkyl group having 1 to 6 carbon atoms optionally substituted with 1 to 3 fluorine atoms, hydroxyl group, an alkoxy group having 1 to 6 carbon atoms optionally substituted with 1 to 3 fluorine atoms, phenyl group, amino group, an alkylamino group having 1 to 6 carbon atoms, carbamoyl group, sulfamoyl group, an acyl group having 2 to 6 carbon atoms, cyano group and an alkynyl group having 3 to 6 carbon atoms;
Y is a group selected from hydrogen atom, an alkyl group having 1 to 6 carbon atoms, an alkenyl group having 3 to 6 carbon atoms, an alkynyl group having 3 to 6 carbon atoms, a cycloalkyl group having 3 to 6 carbon atoms, an alkyl group having 1 to 3 carbon atoms substituted with a cycloalkyl group having 3 to 6 carbon atoms, adamantyl group, phenyl group, naphthyl group, and an alkyl group having 1 to 3 carbon atoms substituted with an aryl group;
R₆ is hydrogen atom, an alkyl group having 1 to 6 carbon atoms, a cycloalkyl group having 3 to 6 carbon atoms, an alkyl group having 1 to 3 carbon atoms substituted with a cycloalkyl group having 3 to 6 carbon atoms, an alkenyl group having 3 to 6 carbon atoms, phenyl group, an alkyl group having 1 to 3 carbon atoms substituted with phenyl group, a hydroxyalkyl group having 2 to 6 carbon atoms, an acyl group having 2 to 6 carbon atoms or benzoyl group;
Y and A may combine to form oxo or hydroxylimino group; and
R₁ and R₂, which may be the same or different, represent hydrogen atom, an alkyl group having 1 to 3 carbon atoms, a cycloalkyl group having 3 to 6 carbon atoms, a hydroxyalkyl group having 2 or 3 carbon atoms, or an alkenyl group having 3 to 6 carbon atoms.

Among the aforementioned preferred compounds, particularly preferred compounds of the present invention include those wherein:
R₃ is an alkyl group having 1 to 3 carbon atoms,
p is an integer of 3-6
R₄ and R₅ are hydrogen atoms;
X' is -S-, -O-, or -N(R₁₁)- wherein R₁₁ is hydrogen atom, an alkyl group having 1 to 6 carbon atoms, or an acyl group having 2 to 6 carbon atoms;
X represents a cycloalkyl group having 3 to 6 carbon atoms, an alkyl group having 1 to 3 carbon atoms substituted with a cycloalkyl group having 3 to 6 carbon atoms, a cycloalkyl group having 3 to 6 carbon atoms substituted with an alkyl group having 1 to 3 carbon atoms, adamantyl group, an aryl group or a heteroaryl group, wherein the aryl group and the heteroaryl group may be substituted with 1 to 3 substituents independently selected from the group consisting of a halogen atom, nitro group, a cycloalkyl group having 3 to 6 carbon atoms, an alkenyl group having 3 to 6 carbon atoms, an alkyl group having 1 to 6 carbon atoms optionally substituted with 1 to 3 fluorine atoms, hydroxyl group, an alkoxy group having 1 to 6 carbon atoms optionally substituted with 1 to 3 fluorine atoms, phenyl group, amino group, an alkylamino group having 1 to 6 carbon atoms, carbamoyl group, sulfamoyl group, an acyl group having 2 to 6 carbon atoms, cyano group and an alkynyl group having 3 to 6 carbon atoms;
Y is hydrogen atom or an alkyl group having 1 to 6 carbon atoms;
R₆ is hydrogen atom, an alkyl group having 1 to 6 carbon atoms, or an acyl group having 2 to 6 carbon atoms;
Y and A may combine to form oxo or hydroxylimino group; and
R₁ and R₂, which may be the same or different, represent hydrogen atom, or an alkyl group having 1 to 3 carbon atoms.

More specifically, each of compounds wherein:
both of R₁ and R₂ are hydrogen atoms, and R₃ is methyl group;
R₁, R₂, R₄, and R₅ are hydrogen atoms;
p is 4;
A is hydroxy group, methoxy group or acetoxy group;
Y is hydrogen atom or methyl group;
A together with Y represent oxo group or hydroxyimino group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and/or
B is a group represented by: 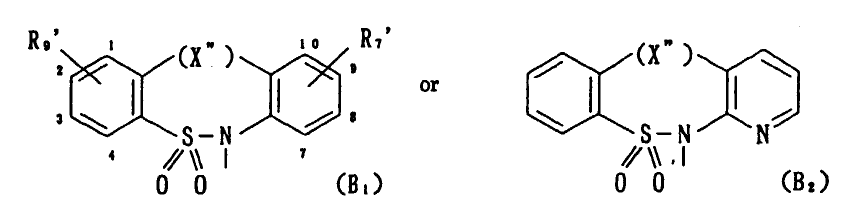
wherein X'' represents -O-, -S-,-NH-, -N(CH₃)- or -N(COCH₃)-; R₇' represents hydrogen atom, 8-F, 9-F, 8-Cl or 8-OCH₃; and R₉' represents hydrogen atom or 3-Cl (indication "8-F" and other represent, for example, that fluorine atom substitutes at 8-position of the nucleus) are particularly preferred compounds of the present invention.

Furthermore, the following compounds are particularly preferred:
(1) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 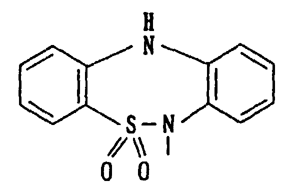
(2) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 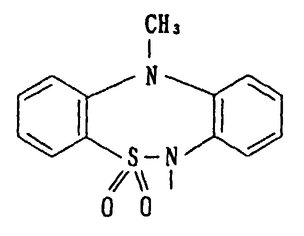
(3) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 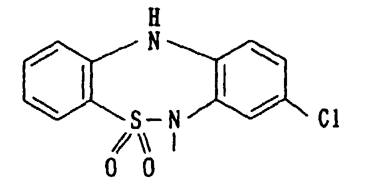
(4) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A and Y may form oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 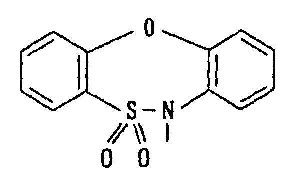
(5) compounds wherein:
   both of R, and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 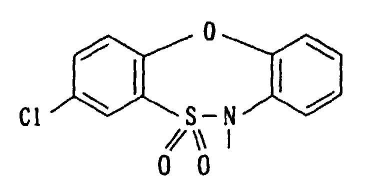
(6) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 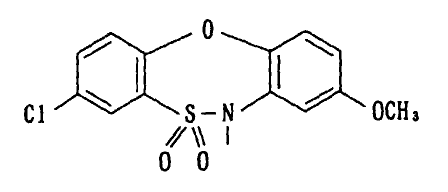
(7) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 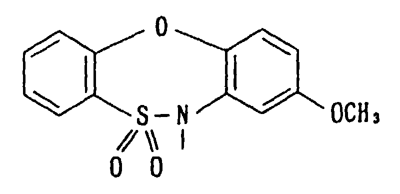
(8) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 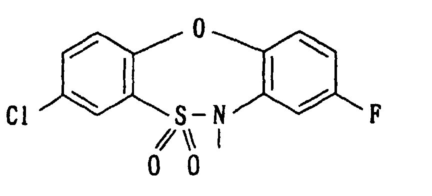
(9) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula: 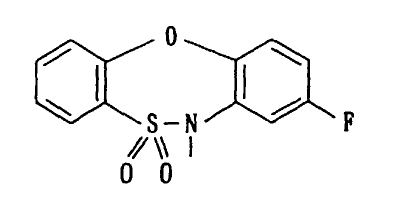 and
(10) compounds wherein:
   both of R₁ and R₂ are hydrogen atoms, and R₃ is methyl group;
   R₁, R₂, R₄ and R₅ are hydrogen atoms;
   p is 4;
   A is hydroxy group;
   Y is hydrogen atom;
   A together with Y represent oxo group;
   X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
   B is represented by the following formula.

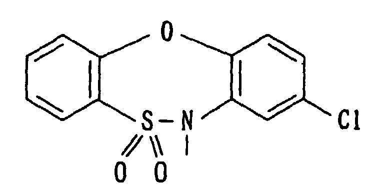

Specific examples of the compounds of the present invention are listed below. In the table, Me represents methyl group, Ada represents adamantyl group, Ph represents phenyl group, cHex represents cyclohexyl group, Naph represents naphthyl group, and Ac represents acetyl group, and 4-Cl-Ph, 4-OMe-Ph, 4-Me-Ph, 4-CF₃-Ph, 4-OCF₃-Ph, 4-H₂NSO₂-Ph, 4-CN-Ph, 4-NO₂-Ph, 4-OH-Ph, 2,4-diF-Ph, 3-F-Ph, 3-Cl-Ph, 3-Me-Ph, 3-OMe-Ph, 2-F-Ph, 2-Cl-Ph, 2-Me-Ph and 4-F-Ph represent 4-chlorophenyl group, 4-methoxyphenyl group, 4-methylphenyl group, 4-trifluoromethylphenyl group, 4-trifluoromethoxyphenyl group, 4-aminosulfonylphenyl group, 4-cyanophenyl group, 4-nitrophenyl group, 4-hydroxyphenyl group, 2,4-difluorophenyl group, 3-fluorophenyl group, 3-chlorophenyl group, 3-methylphenyl group, 3-methoxyphenyl group, 2-fluorophenyl group, 2-chlorophenyl group, 2-methylphenyl group and 4-fluorophenyl group, respectively.

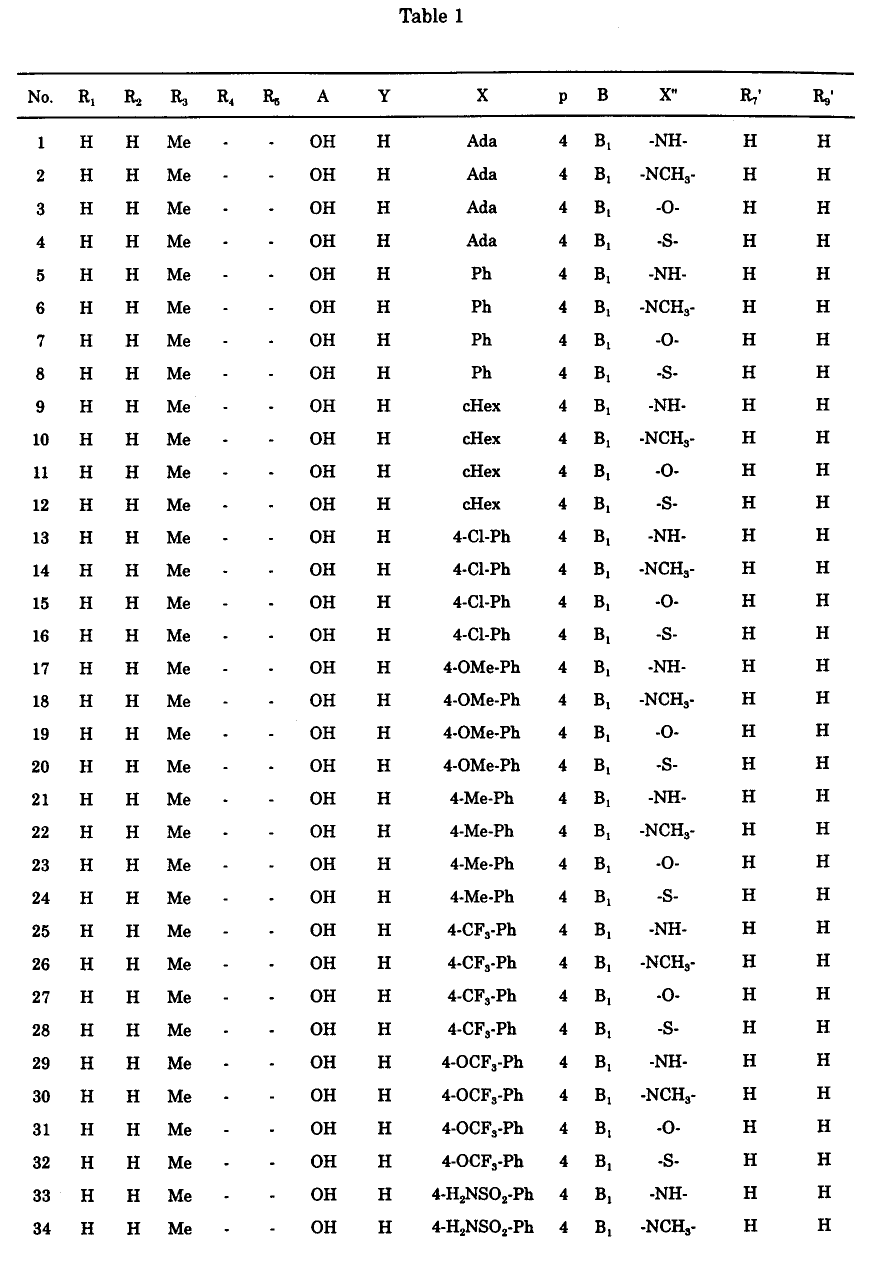

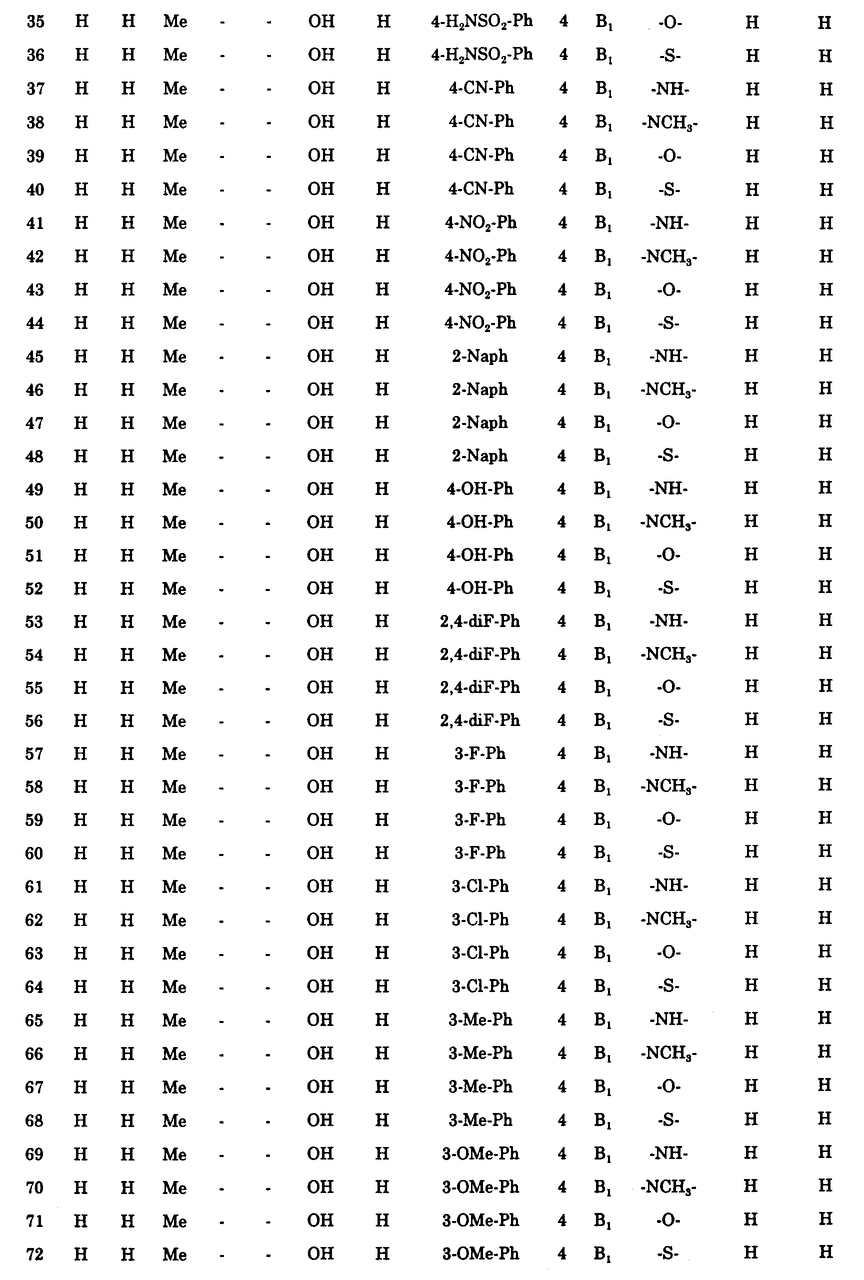

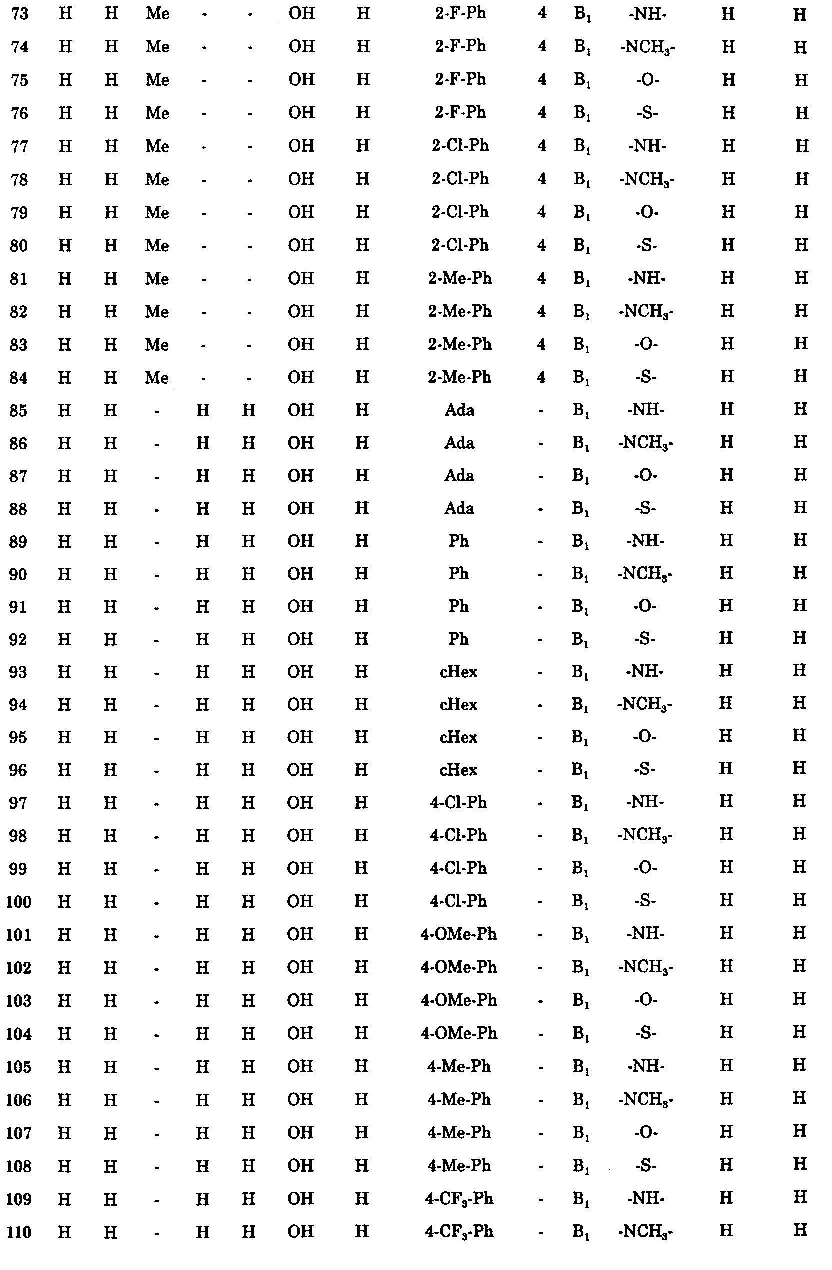

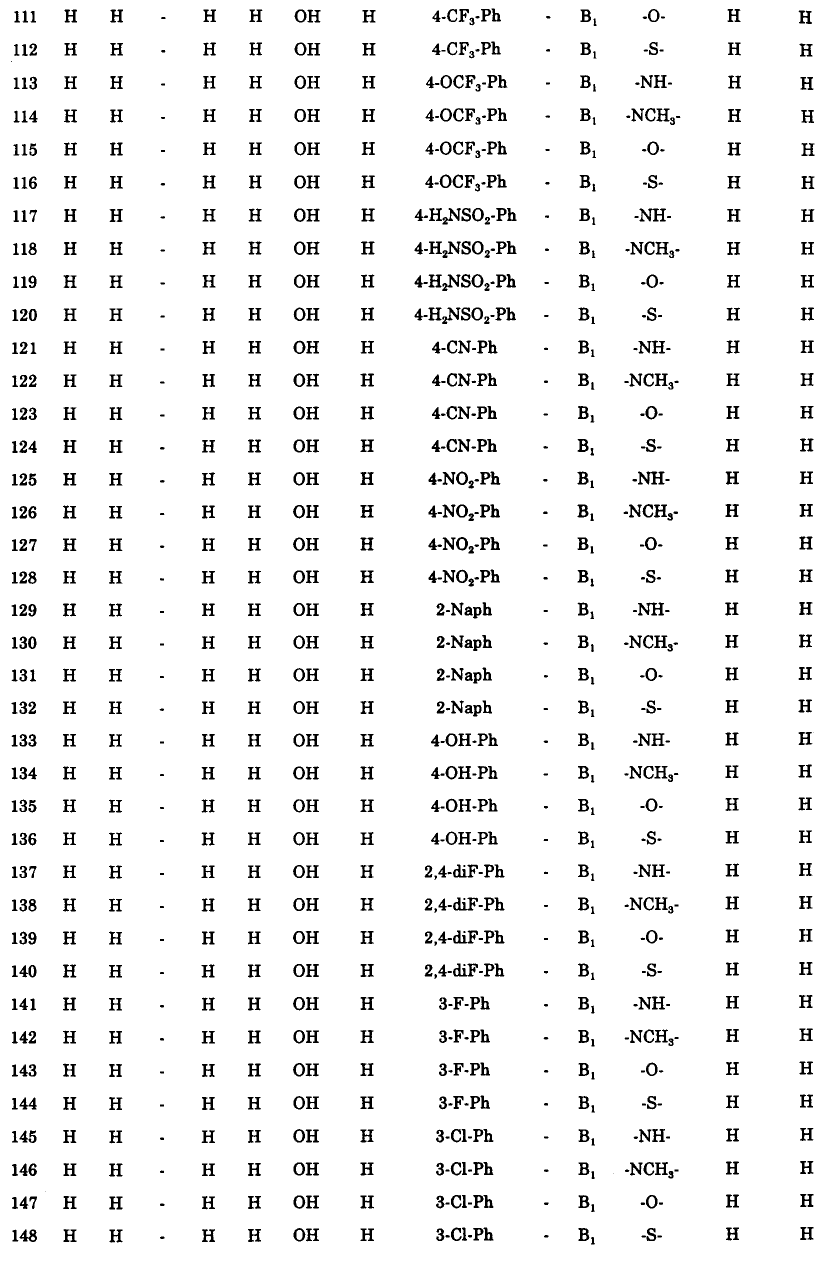

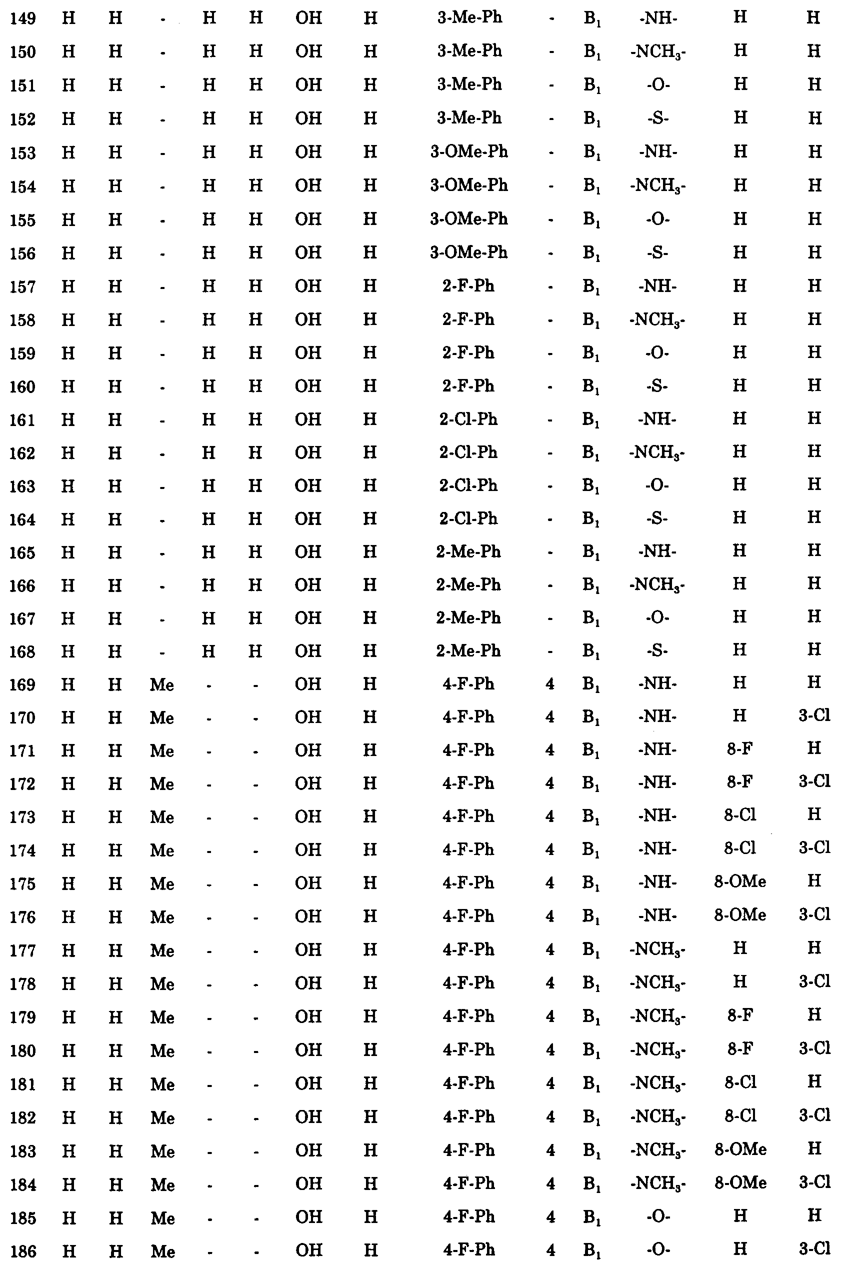

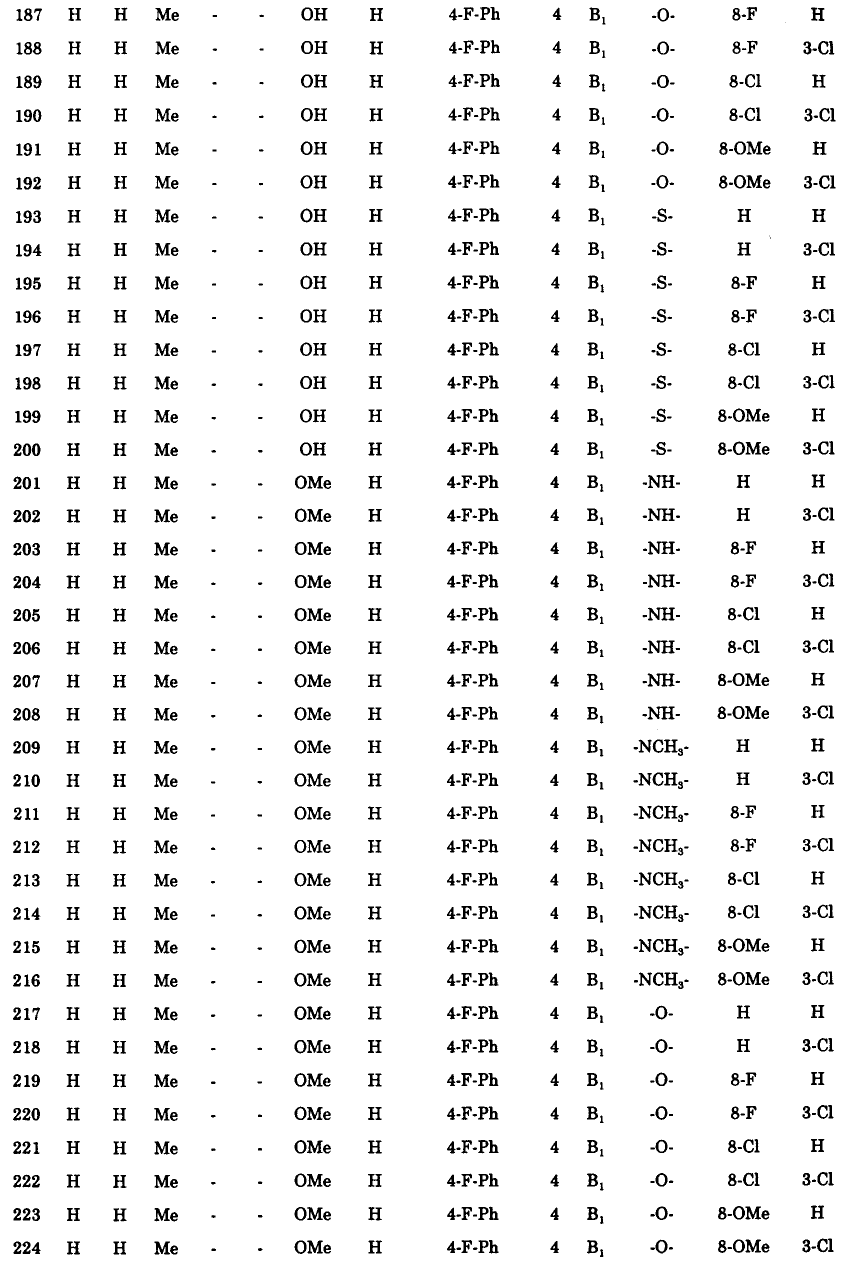

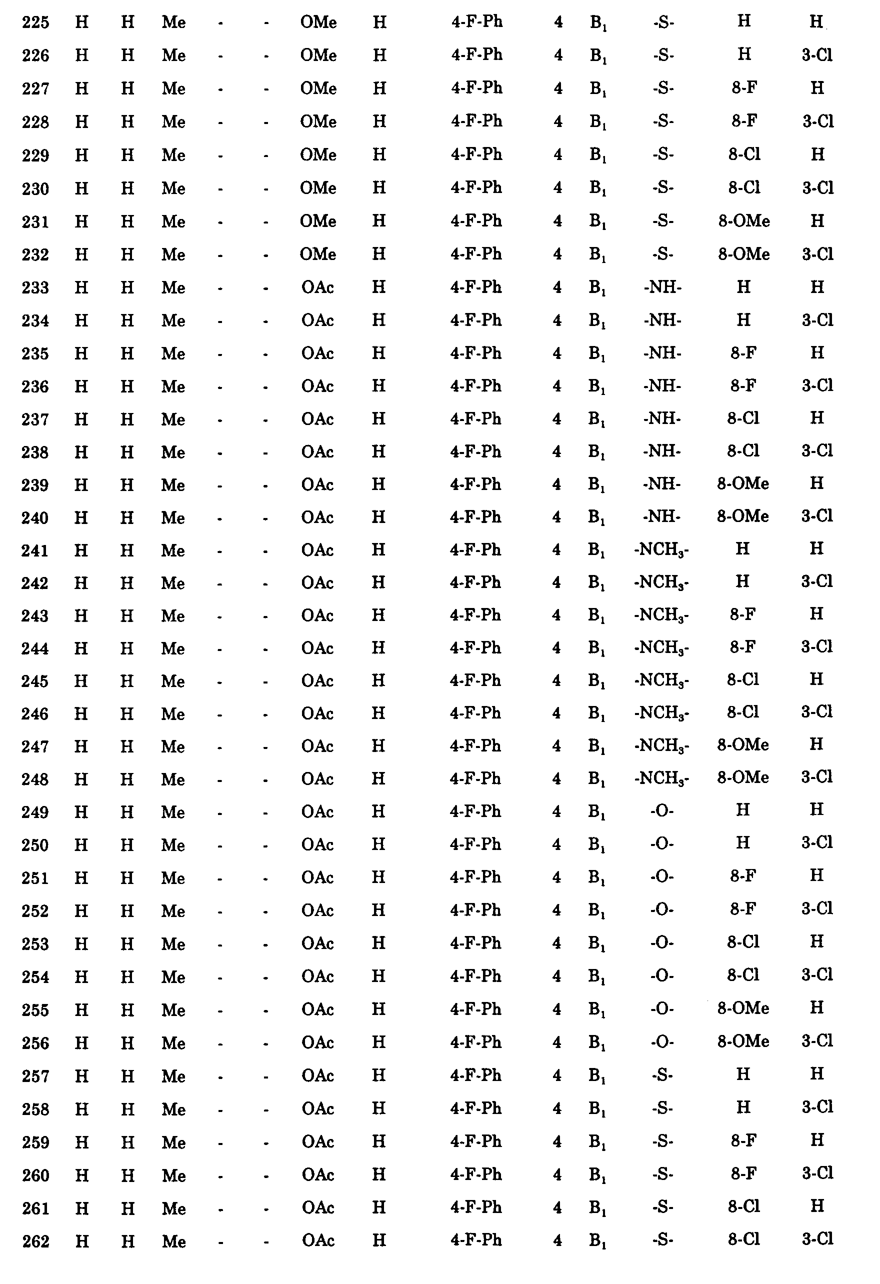

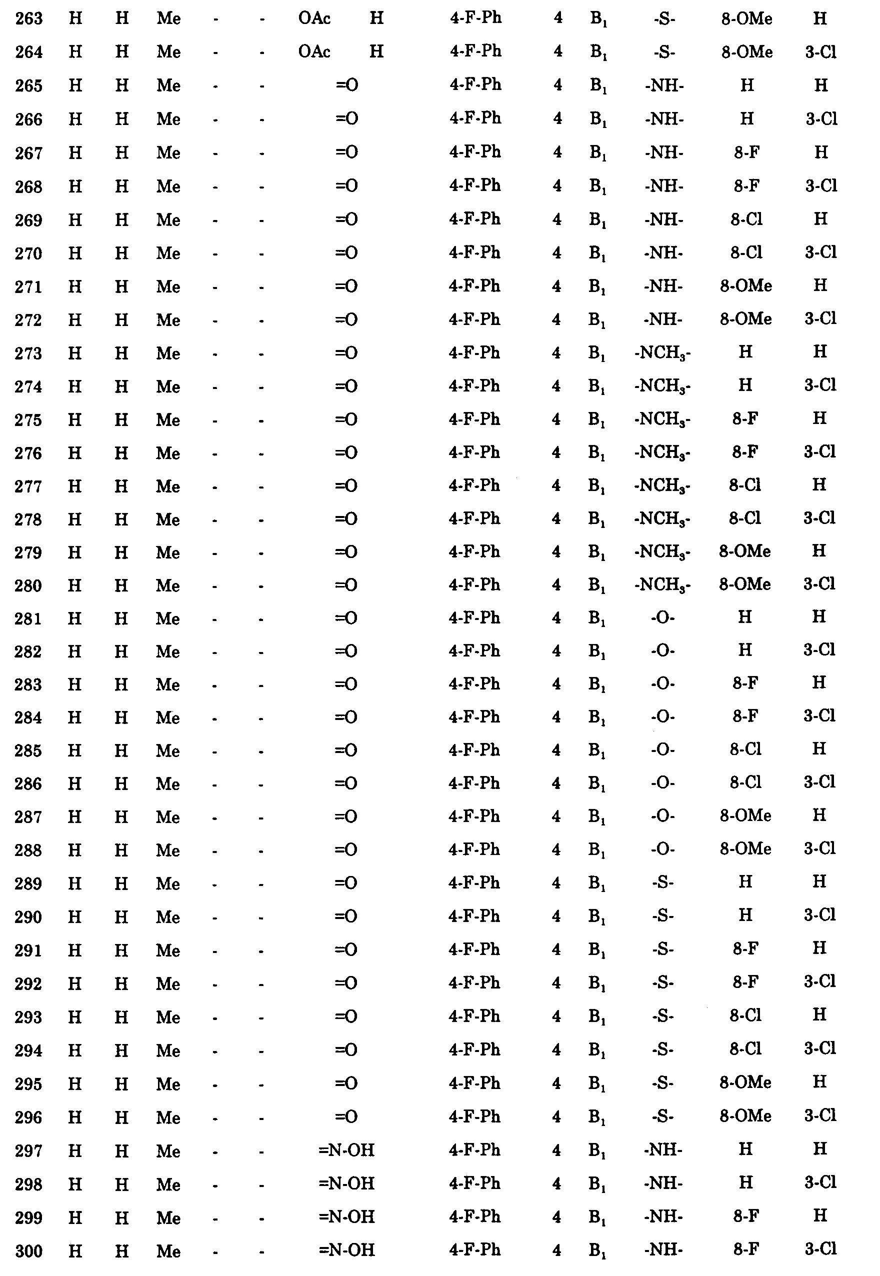

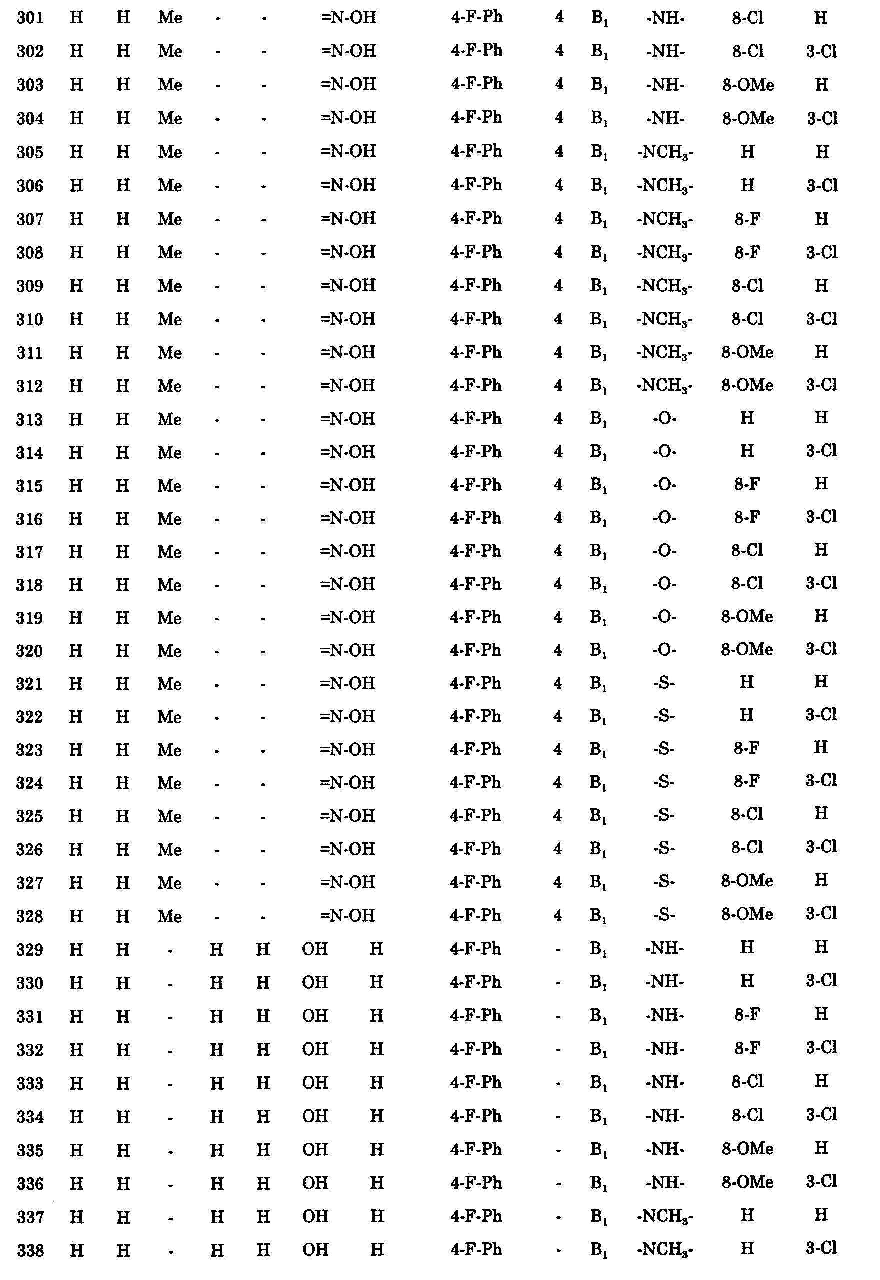

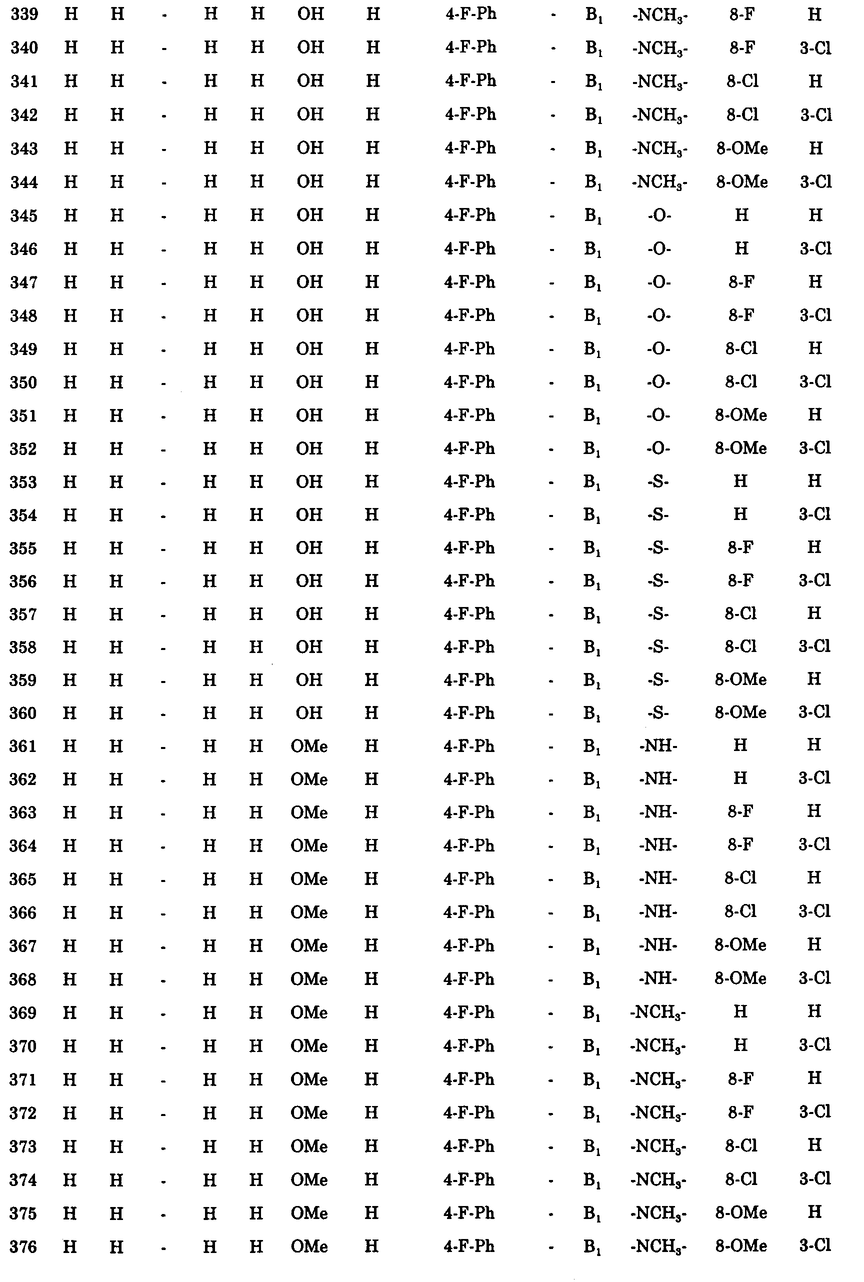

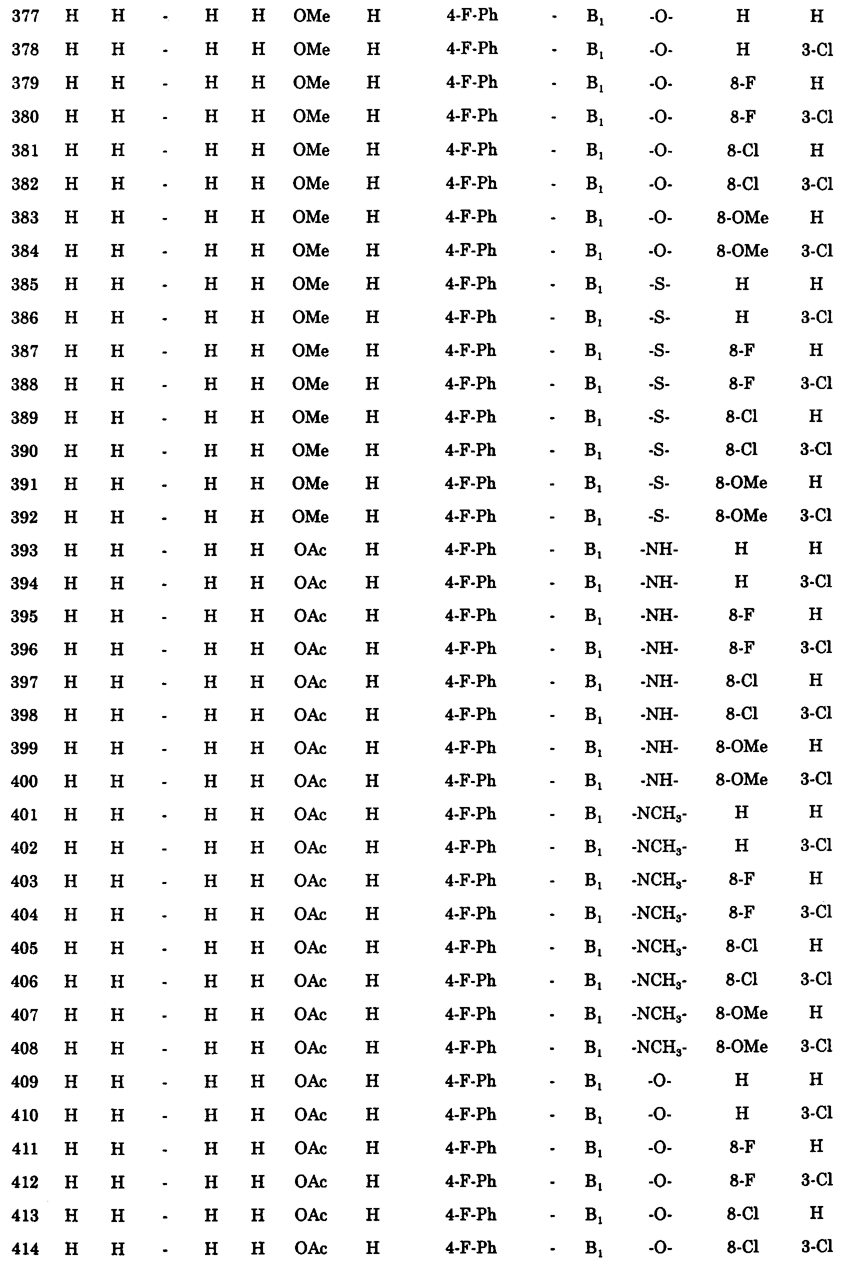

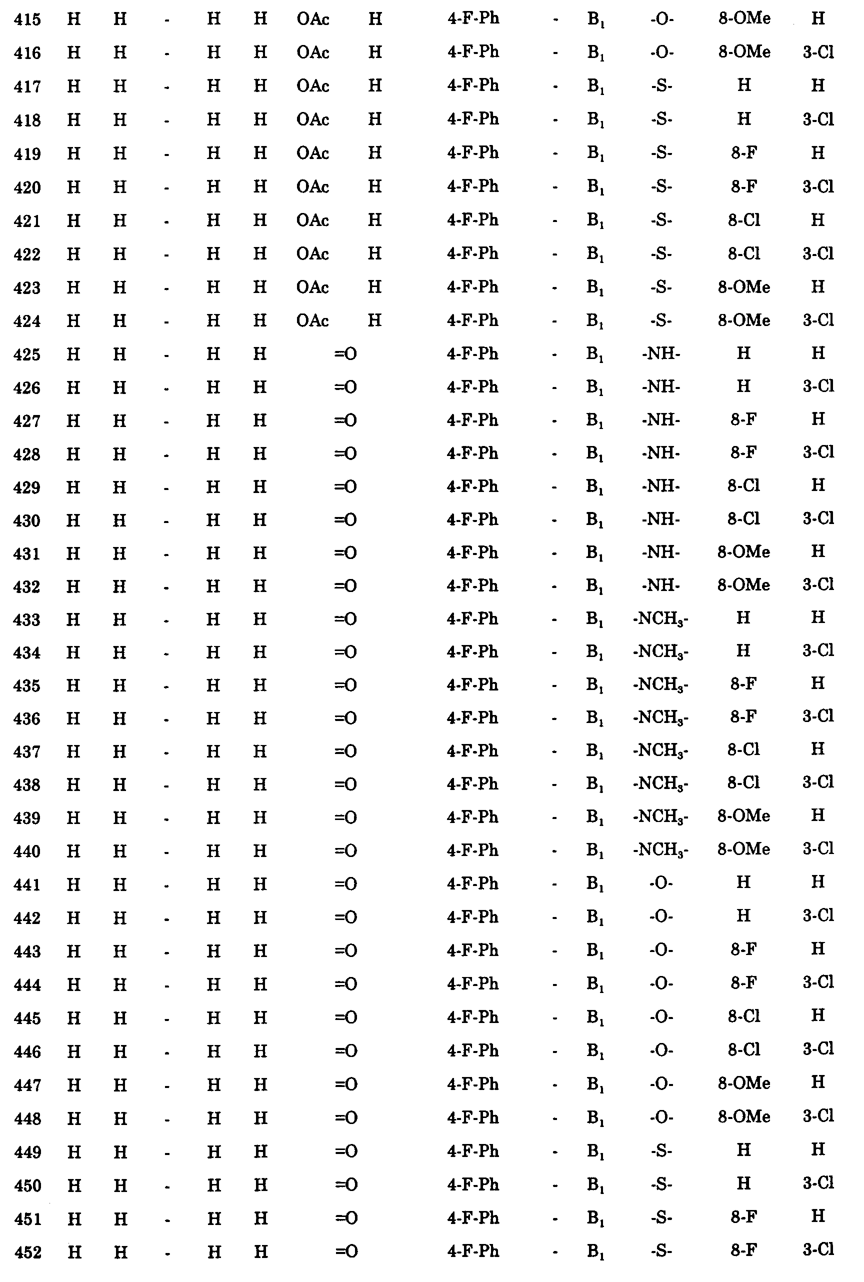

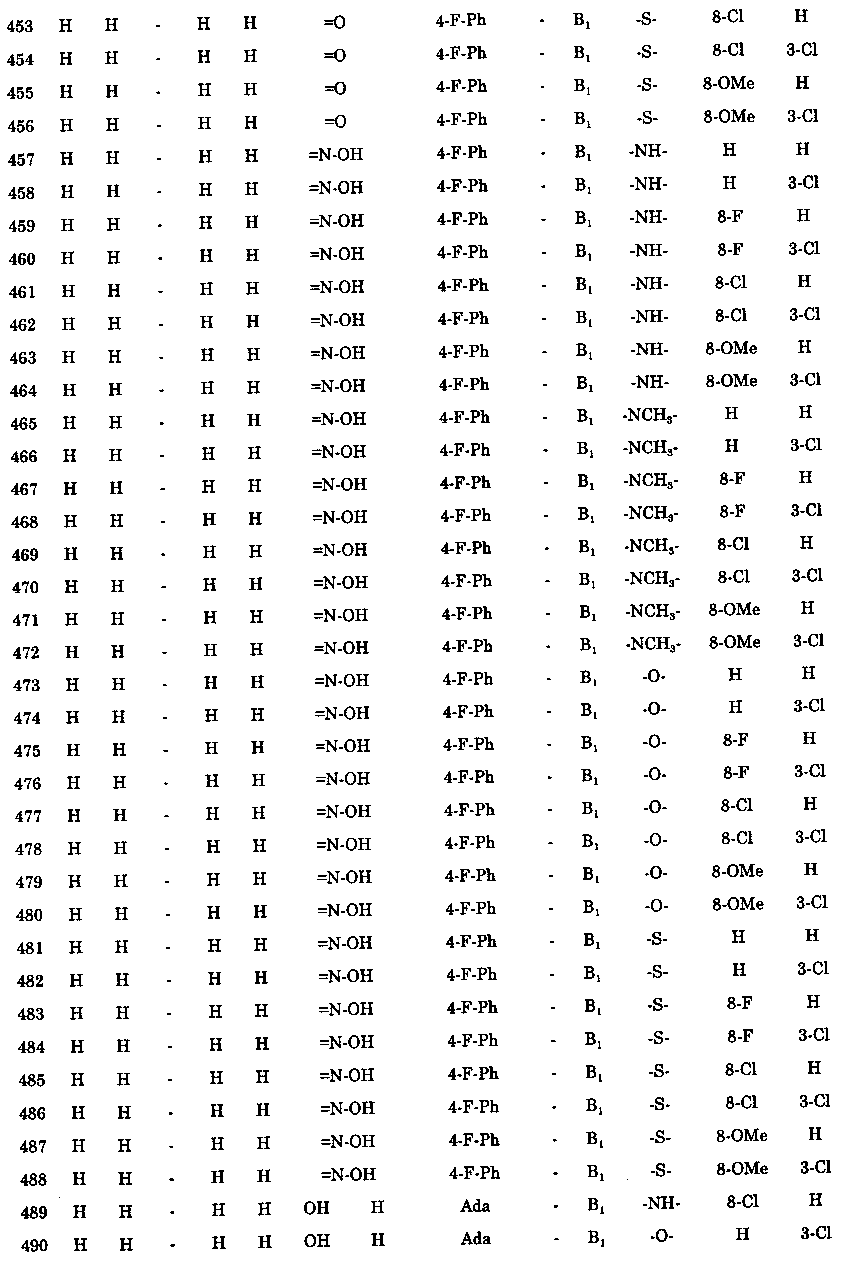

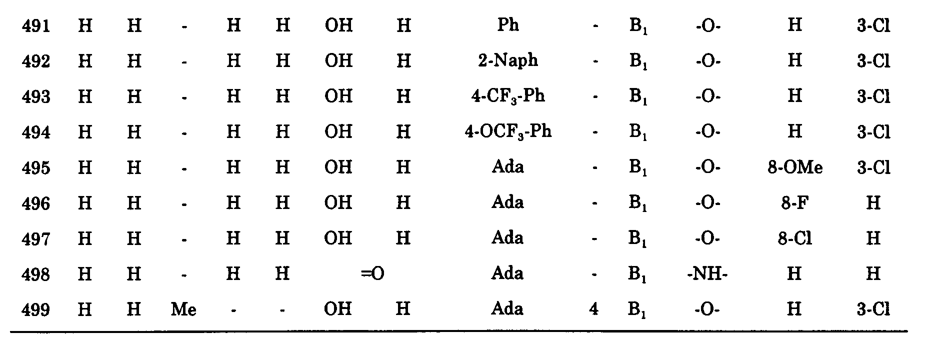

Specifically, compounds of Examples 2, 3, 10, 16, 18, 19, 23 and 24, which will be explained below, are particularly preferred compounds of the present invention.

However, the scope of the present invention is not limited to the aforementioned preferred compounds and particularly preferred compounds mentioned above, as well as to the compounds specifically mentioned above.

The compounds of the present invention can exist as optical isomers, and both the racemates and individual optical isomers fall within the scope of the present invention. The racemates can be separated into their individual isomers by methods well-known to those skilled in the art.

Acid addition salts, preferably pharmacologically acceptable addition salts, hydrates and any solvates of the compounds of the present invention represented by the aforementioned formula also fall within the scope of the present invention.

Examples of the salts which can be formed by the compounds of the present invention include, for example, inorganic acid salts such as hydrochlorides, hydrobromides, hydriodides, sulfates, nitrates and phosphates, organic acid salts such as succinates, acetates, glycolates, methanesulfonates, and toluenesulfonates, alkali metal salts such as sodium salts, and potassium salts, alkaline earth metal salts such as magnesium salts, and calcium salts, ammonium salts such as ammonium salts, and alkylammonium salts.

Examples of solvents of the solvates which can be formed by the compounds of the present invention include, for example, methanol, ethanol, isopropanol, acetone, and ethyl acetate.

The compounds of the present invention have high affinity and selectivity for the sigma 2 binding site. Accordingly, the compounds of the present invention are useful as medicaments for therapeutic and/or preventive treatment of various diseases and/or conditions in mammals including human, preferably in human, which are affected or facilitated by the neuromodulatory effect of sigma 2 ligands. Examples of such diseases include, for example, central nervous system diseases, gastrointestinal diseases and cardiovascular system diseases.

Examples of the central nervous system diseases include, for example, anxiety, depression or dysthymic disorders, psychosis, narcotic drug abuse, pain, dyskinesia, cerebral vascular disorders, epilepsy, dementia including Alzheimer's disease, Parkinsonism, nueropathological disorders and the like. Examples of the gastrointestinal diseases include, for example, irritable bowel syndrome, irritable colon, spastic colon, mucous colitis, acute enteroccolitis, diverticulitis, dysentery and the like. Examples of the cardiovascular system diseases include, for example, hypertension, arrhythmia, angina and the like. However, indications of the medicament of the present invention are not limited to these specific diseases and/or symptoms, and the medicament is applicable to therapeutic and/or preventive treatment of variety of diseases and/or symptoms in which sigma 2 ligands participate in vivo.

The present invention also provide pharmaceutical compositions comprising the compound of the present invention as an active ingredient in an amount of from about 0.01 to 100 mg per unit dose. The pharmaceutical compositions may be administered by any suitable administration route. For example, they may be administered orally in the form of tablets, capsules or the like, intravenously in the form of solutions for injection or the like, topically in the form of ointments, lotions or the like, or ocularly in the form of eye drops or the like. For the manufacture of the pharmaceutical compositions mentioned above, methods and pharmaceutical additives well-known in the art may be used, and pharmaceutical compositions having suitable properties depending on the desired route of administration can be manufactured. The daily dose for an adult usually ranges from about 0.05 to 500 mg.

The compounds of the present invention represented by formula (I) may be prepared by general route of synthesis as described in the following methods.

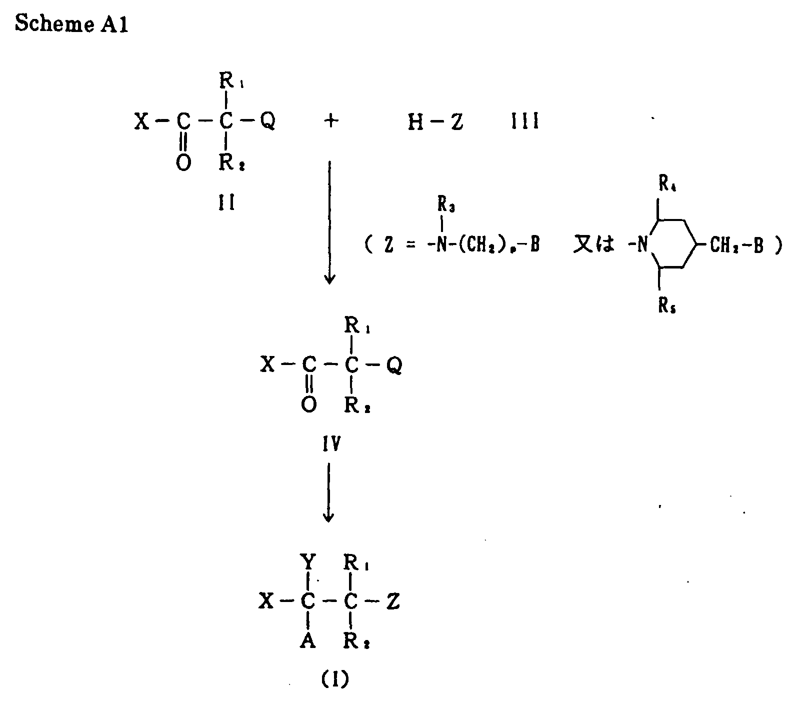

### Method A:

A compound of formula (II), wherein Q is a halogen atom such as chlorine atom, bromine atom, and iodine atom, or tosylate, mesylate or the like, may be reacted with a nucleophilic amino derivative represented by formula (III): H-Z to yield a corresponding compound of formula (IV). The reaction may typically be conducted in the presence of a base such as triethylamine and potassium carbonate in a polar solvent such as dimethylformamide and acetonitrile. The amino-keto derivative of formula (IV) can be reduced to give a hydroxy derivative of formula (I) wherein A=OH and Y=H. The reduction may generally be accomplished performed using sodium borohydride in ethanol, methanol or tetrahydrofuran at room temperature. The amino-keto derivative can also be reacted with an organometal reagent such as Y-MgBr to give a compound of formula (I) wherein A=OH.

The acyloxy and alkoxy compounds can be prepared by conventional methods starting from the free hydroxy derivative. The O-alkyl derivatives may be prepared by solvolysis of a sulfonyl ester intermediates [Advanced Organic Chemistry, J. March, John Wiley & Sons, New York, 1985, 264-317]. Chiral ethers can also be obtained by solvolysis of chiral sulfonyl ester derivatives such as camphorsulfonate. The oximes of the keto derivatives of formula (IV) can be prepared according to an oximation as described in Organic Functional Group Preparation Vol. III, S. R. Sandler and W. Karo, Academic Press, London, 1989, 430-481.

The synthesis of the compound of formula (III), as the starting material mentioned in Scheme A1, is depicted in Schemes A2, A3, and A4.

In the schemes, piperidine type compounds are chosen as the target amino derivative of formula (VII); however, acyclic amino derivatives can also be obtained according to the same process. The P substituent in the compounds of formulas (V) and (VII) represents an amino protective such as those described in Protective Groups in Organic Synthesis [T. W. Greene and P. G. M. Wuts, John Wiley & Sons, New York, 1991]. Such protective group is easily removed to give the piperidine compound of formula (III).

N-Alkylation of non-cyclized intermediate of formula (VI) or (VIII) can be conducted by using activated derivatives of formula (V) wherein Q is a halogen atom such as chlorine atom, bromine atom and iodine atom, or tosylate, mesylate or the like. The N-alkylation reaction may preferably be conducted in dimethylformaldehyde in the presence of sodium hydride as a base and at a temperature above 50°C. If desired, up to 1 molar equivalent of iodide salt (e.g., NaI, KI) may be added to the reaction as a catalyst. The reaction can also be conducted under phase-transfer process using toluene as a solvent and sodium hydroxide or potassium hydroxide as a base in the presence of a catalytic amount of tetrabutylammonium hydrogen sulfate or other suitable salts.

N-Alkylation of the heterocyclic rings can proceed directly through the Mitsunobu reaction (O. Mitsunobu, Synthesis, 1981, 1-28) from the hydroxyl derivative of formula (V) wherein Q=OH.

Scheme A2 depicts the synthesis of dibenzothiadiazepine derivatives.

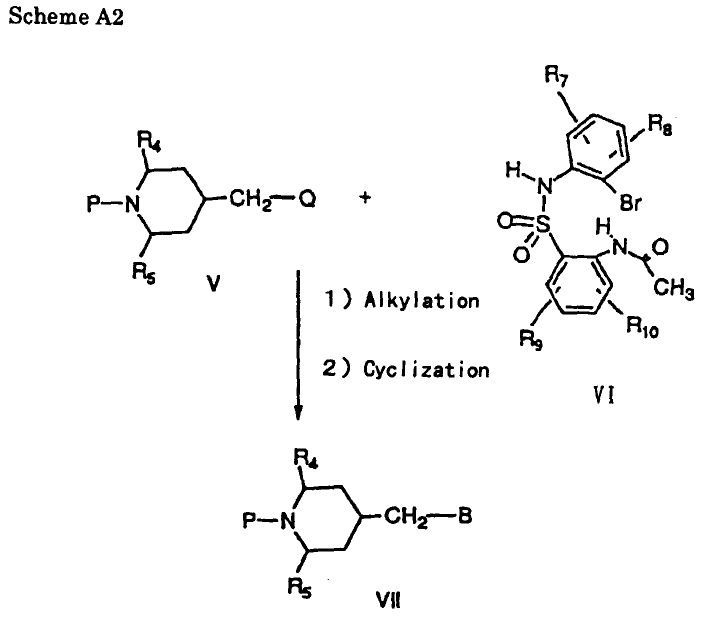

The intramolecular cyclization for nitrogen nucleophiles can be carried out according to the Ullmann amine arylation [F. Ullmann, Ber. Chem. Ges., 1903, 36, 2382; R. G. R. Bacon and H. A. O. Hill, Q. Rev. Chem. Soc., 1965, 19, 95] or to the Goldberg amide arylation [I. Goldberg, Ber. Dtsch Chem. Ges., 1906, 39, 1691] and its variants [A. Greiner, Synthesis, 1989, 312-313].

Similar to the method of Scheme A2, Scheme A3 depicts the method for preparing the target amino derivatives of formula (VII) by using an oxo compound of formula (VIII). In the scheme, the synthesis of dibenzoxathiazepine derivatives is shown. Q 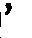 represents chlorine atom or bromine atom. The ring closure of the hydroxy or acetoxy intermediates can be conducted according to, for example, a method described in literature [Belg. Patent 622185; K. Nagarajan, V. Ranga Rao, A. Venkateswarlu and R. K. Shah Indian J. Chem., 1974, 12, 252-257], preferably in a polar solvent such as dimethylformamide or N-methylpyrrolidone at a temperature ranging from 120°C to 160°C using sodium ethoxide or sodium methoxide as a base.

The ring closure can also be carried out in water [R. F. Pellon, R. Carrasco, V. Milian and L. Rodes, Synth. Commun., 1995, 25(7), 1077-1083].

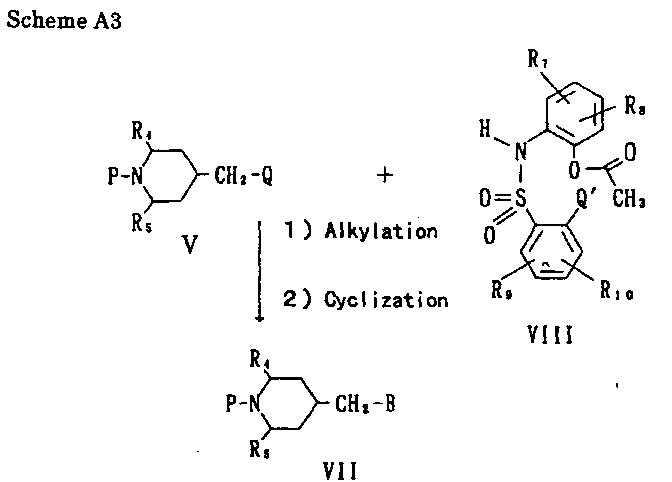

The dibenzodithiazepine derivatives of the general formula (VII) can be prepared according to a similar method using an acylthio derivative of the general formula (VI) or (VIII).

As shown in Schemes A4, the N-alkylation of free diphenylsultam derivatives of formula (IX) can also be conducted by a method similar to those described in Schemes A2 and A3.

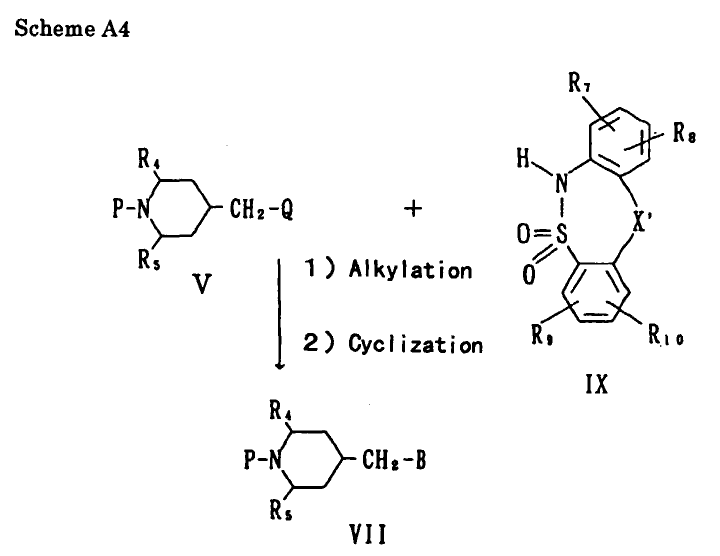

The free diphenylsultams of formula (IX) are generally obtained by preparing a sulfamide derivative according to a known method, and then cyclizing the sulfamide derivative under the conditions shown in Schemes A2 and A3. Functional groups may be converted before the cyclization as required. For example, an amino precursor is diazotized and then the resultant is reacted with potassium thiocyanate and further reduced to a thiol derivative to obtain thiolsulfonamide intermediates [G. T. Whiteker and S. J. Lippard, Tetrahedron Letters, 1991, 32, 38 5019-5020]. The thiol intermediate can be cyclized to obtain the dibenzodithiazepine compounds.

The thiolsulfonamide intermediates mentioned above can also be prepared according to the phenol/thiophenol conversion [C. Corral, J. Lissavetzky and A. M. Valdeomillos, Synthesis, 1983, 172; Sebok et al., J. Org. Chem., 1994, 59, 21, 6318-6321].

The dibenzooxathiazepine ring may also be prepared according to other synthetic routes [R. A. Abramovitch, C. I. Azogu and I. T. McMaster, J. Am. Chem. Soc. 1969, 91, 5, 1219-1220; R. A. Abramovitch, C. I. Azogu, I. T. McMaster and D. P. Vanderpool, J. Org. Chem., 1978, 43, 6, 1218-1226; P. S. Dewar, A. R. Forrester and R. H. Thomson, J. Chem. Soc. Perkin 1, 1972, 2862-2865].

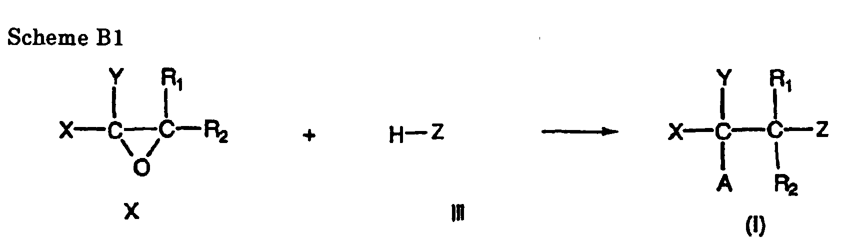

### Method B

Another route for the preparation of the compounds of formula (I) wherein A is -OH is outlined in Scheme B. The aminolysis of epoxy derivatives is generally conducted by heating the reactives in a solvent such as ethanol [N. S. Isaacs and R. E. Parker, J., 1960, 3497-3504]; aminolysis reaction may be performed by using metal salts as catalysts [M. Chini, P. Crotti and F. Macchia, Tetrahedron Letters, 1990, 31,32, 4661-4664]. The epoxy derivatives of formula (X) can be obtained according to oxidation of alkenyl derivatives, dehydrohalogenation of halohydrin compounds, or reaction of a carboxyaldehyde with dimethylsulfonium methylide [T. Kutsuma, I. Nagayama, T. Ozaki, T. Sakamoto, S. Akaboshi, Heterocycles, 1977, 8, 397-401]. When the starting epoxy derivative of formula (X) is used in an optically active form, the amino alcohol of formula (I) can be obtained in an optically active form.

The compounds used the starting materials in the above-mentioned processes may be in racemic form or in the desired optically active form to give the corresponding racemic or optically active compounds. The desired optically active enantiomer may also be obtained by resolution according to conventional techniques. The compounds of formula (I) which are basic in nature can form acid addition salts, preferably pharmacologically acceptable salts, with various inorganic and organic acids. These salts can readily be prepared by treating the base compounds of formula (I) with a substantially equivalent amount of the chosen mineral or organic acids in a suitable organic solvent such as methanol, ethanol, isopropanol and ethyl acetate.

### Examples

The present invention will be further explained more specifically by referring to the following examples. However, the scope of the present invention is not limited to these examples.

### Example 1: 6-[[1-[2-(1-Adamantyl)-2-oxoethyl]-4-piperidinyl]methyl]-6,11-dihydrodibenzo[c,f][1,2,5]thiadiazepine 5,5-dioxide·hydrochloride (hydrochloride of Compound No. 498 shown in Table 1)

### a) Piperidine-1,4-dicarboxylic acid, 1-tert-butyl ester

Di-tert-butyl dicarbonate (101.1 g, 464 mM) was added dropwise to a solution of isonipecotic acid (60 g, 464 mM) and sodium hydroxide (37.6 g, 940 mM) in water (86 ml) and tert-butyl alcohol (176 ml) with stirring. After the end of the addition, the above mixture was added with tert-butyl alcohol (100 ml), and stirred at room temperature for three hours. The resulting solution was diluted with water (200 ml), and extracted twice with 150 ml of pentane. The aqueous layer was acidified with 70 g of potassium bisulfate with cooling, and extracted with ethyl acetate. Standard workup gave the title compound as a white powder (102.3 g. yield 96%). Melting point: 144-146°C.

### b) tert-Butyl 4-(hydroxymethyl)piperidine-1-carboxylate

To a solution of the compound obtained in Example 1(a) (13.74 g, 60 mM) in 1,2-dimethoxyethane (60 ml), N-methylmorpholine (6.66 ml, 60 mM) and isobutyl chloroformate (8.16 ml, 60 mM) were added successively under cooling (-15°C). After 10 minutes, the precipitates were removed by filtration, and washed with 60 ml of 1,2-dimethoxyethane. To the filtrate on an ice-salt bath, 30 ml of aqueous sodium borohydride (3.42 g, 90 mM) was carefully added. After the end of the addition, the mixture was stirred for 45 minutes, and then added with 800 ml of water. The expected alcohol compound was extracted with ethyl acetate (400 ml), and the organic layer was washed successively with 0.05 N hydrochloric acid, water, and saturated aqueous sodium bicarbonate. After drying, the solvent was evaporated under reduced pressure to obtain the title compound as a colorless oil (9.8 g, yield: 75%). By standing at room temperature, the oil crystallized as white crystals (melting point: 74-76°C).

### c) tert-Butyl 4-(methanesulfonyloxymethyl)piperidine-1-carboxylate

To a solution of the compound obtained in Example 1(b) (26.8 g, 125 mM) and triethylamine (19.1 ml, 137.5 mM) in dry tetrahydrofuran (350 ml), methanesulfonyl chloride (11.3 ml, 137.5 mM) was added dropwise with ice cooling. After stirring for 20 minutes, the reaction mixture was added with water and extracted with ethyl acetate. The organic layer was washed with saturated aqueous sodium bicarbonate, and subjected to a standard workup procedure to obtain the quantitative title compound as a white solid (melting point: 72-74°C).

### d) tert-Butyl 4-[N-(2-(acetylaminophenylsulfonyl))-N-(2-bromophenyl)aminomethyl]-piperidine-1-carboxylate

N-(2-bromophenyl)-2-acetylaminophenylsulfonamide (8.3 g, 22.5 mM) was dissolved in 22 ml of dimethylformamide, and the solution was added to a suspension of sodium hydride (60% content in oil, 0.9 g, 22.5 mM) in 60 ml of dimethylformamide. The reaction mixture was stirred under nitrogen atmosphere at room temperature for 20 minutes, and then added with the above methanesulfonate (6.59 g, 22.5 mM) in dimethylformamide (50 ml) and sodium iodide (1.01 g, 6.75 mM), and the mixture was heated at 80°C for 25 hours. After cooling, the reaction mixture was poured into water, and extracted with ethyl acetate. The standard workup gave brownish oil which was applied to silica gel column chromatography (eluent: hexane/ethyl acetate=7:3) to obtain the title compound as an oil in 64% yield. Rf=0.3 (ethyl acetate/hexane=1:1).

### e) tert-Butyl 4-[6-(6,11-dihydrodibenzo[c,f][1,2,5]thiadiazepine)methyl]piperidine-1-carboxylate S,S-dioxide

To a solution of the compound obtained in Example 1(d) (8.13 g, 14.37 mM) in dimethylformamide (60 ml), potassium carbonate (1.99 g, 14.37 mM), copper powder (0.46 g, 7.24 mM) and copper (I) bromide (0.21 g, 1.46 mM) were added. The reaction mixture was heated under reflux for 21 hours with stirring. After cooing, the reaction mixture was filtered, and the filtrate was diluted with water. The precipitates were collected, washed with water, and dried to obtian the title compound as a pale purple solid (4.15 g, yield: 67%). Melting point: 89-92°C, Rf=0.3 (ethyl acetate/hexane=6:4).

### f) 4-[6-(6,11-Dihydrodibenzo[c,f][1,2,5]thiadiazepine)methyl]piperidine S,S-dioxide

A 4N solution of hydrochloric acid/ethyl acetate (11 ml) was added to a solution of tert-butyl 4-[6-(6,11-dihydrodibenzo[c,f][1,2,5]thiadiazepine)methyl]piperidine-1-carboxylate S,S-dioxide (4 g, 9.03 mM) in ethyl acetate (50 ml). The solution was stirred under reflux for two hours, and then stirred at room temperature for 12 hours. After cooling, the resulting solid was filtered, and washed with ethyl acetate until neutral to obtain hydrochloride of 4-[6-(6,11-dihydrodibenzo[c,f][1,2,5]thiadiazepine)methyl]piperidine S,S-dioxide (2.95 g, 86%). Melting point: 267-270 °C. The hydrochloride was treated with 0.5N aqueous sodium hydroxide, and extracted with methylene chloride. After drying, the solvent was evaporated under reduced pressure to obtain a free compound as a foam (quantitative).

### g) 6-[[1-[2-(1-Adamantyl)-2-oxoethyl]-4-piperidinyl]methyl]-6,11-dihydrodibenzo[c,f][1,2,5]thiadiazepine 5,5-dioxide hydrochloride

A solution of the free compound obtained in Example 1(f) (2.19 g, 6.4 mM), 1-(bromoacetyl)adamantane (1.8 g, 7 mM) and potassium carbonate (1.05 g, 7.6 mM) in acetonitrile (80 ml) was heated under reflux for two hours. After cooling, the mixture was added with water, and extracted with methylene chloride. After drying over sodium sulfate, the solvent was evaporated under reduced pressure, and the resulting residue was purified by silica gel column chromatography (eluent: ethyl acetate/dichloromethane=1:1, Rf=0.35) to obtain the title compound in free form as an orange foam (2.6 g, yield: 78.2%). Two molar excess of 4N hydrochloric acid/ethyl acetate solution was added to the product in ethanol to prepare hydrochloride and the title compound was obtained as a white powdered solid in 72% yield. Melting point: 275-277°C.

| Elemental analysis (C₃₀H₃₈N₃O₃S · HCl · 1/2H₂O) | | | | | |
|---|---|---|---|---|---|
| Calculated: | C, 63.76%; | H, 6.95%; | N, 7.43%; | Cl, 6.27%; | S, 5.67% |
| Found: | C, 63.79%; | H, 6.99%; | N, 7.14%; | Cl, 6.05%; | S, 5.46% |

### Example 2: (R,S)-1-(1-Adamantyl)-2-[4-(6,11-dihydrodibenzo[c,f][1,2,5]thiadiazepin-6-ylmethyl)piperidin-1-yl]ethanol S,S-dioxide hemifumarate (fumaric acid salt of Compound No. 85 shown in Table 1)

To a solution of the free compound obtained in Example 1(g) (2.0 g, 3.8 mM) in ethanol (60 ml), sodium borohydride (718 mg, 19 mM) was added carefully. After the mixture was stirred at room temperature for four hours, the solvent was evaporated under reduced pressure. The resulting oil was added with water, and the product was extracted with methylene chloride. The organic layer was washed twice with saturated aqueous sodium bicarbonate. The organic layer was dried over sodium sulfate, and the solvent was evaporated under reduced pressure to obtain a crude product, which was further purified by silica gel column chromatography (eluent: methanol/dichloromethane=5:95, Rf:0.3) to isolate the title compound in free form as a white solid (1.2 g, yield: 60%). Melting point: 268-270°C. The compound was dissolved in ethanol, and added with fumaric acid to crystallize as hemifumarate from ethanol. White powder, melting point: 203-205°C.

| Elemental analysis (C₃₂H₄₁N₃O₅S · 1/2H₂O) | | | | |
|---|---|---|---|---|
| Calculated: | C, 65.28%; | H, 7.19%; | N, 7.14%; | S, 5.44% |
| Found: | C, 65.63%; | H, 7.24%; | N, 7.01%; | S, 5.37% |

### Example 3: (R,S)-1-(2,4-Difluorophenyl)-2-[4-(6,11-dihydrodibenzo[c,f][1,2,5]thiadiazepin-6-yl-methyl)piperidin-1-yl]ethanol S,S-dioxide hydrochloride (hydrochloride of Compound No. 137 shown in Table 1)

### a) 2,4-Difluorophenyloxirane

A solution of 2-chloro-2 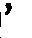 ,4 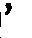 -difluoroacetophenone (2.26 g, 11.9 mmol) in isopropanol (10 ml) was added with sodium borohydride (0.3 g, 8 mmol), and stirred for two hours. Then, the mixture was added with 1N NaOH (10 ml) and stirred at 60°C for two hours. After cooling, the solution was added with water, and extracted with diethyl ether. The organic layer was washed with water, and dried over sodium sulfate, and the solvent was evaporated under reduced pressure. The residue was purified by silica gel column chromatography (eluent: hexane) to obtain the title compound as a colorless oil (890 mg, yield: 48%). Rf=0.85 (ethyl acetate/hexane=1:9).

### b) (R,S)-1-(2,4-Difluorophenyl)-2-[4-(6,11-dihydrodibenzo[c,f][1,2,5]thiadiazepin-6-ylmethyl)piperidin-1-yl]ethanol S,S-dioxide hydrochloride

A solution of the amine obtained in Example 1 (f) (1.4 g, 4.1 mmol) and the epoxide obtained in Example 3(a) (640 mg, 4.1 mmol) in ethanol (20 ml) was heated under reflux for four hours, and after cooling, the solvent was evaporated. Methylene chloride/diethyl ether (1:1) was added to the residue to obtain the title compound in free form (800 mg, yield: 39%). Melting point: 200-202°C.

The above free compound was dissolved in ethanol, and the solution was added with 4N hydrochloric acid/ethyl acetate solution to give the title compound as a white solid in 74% yield. Melting point: 173-175°C.

| Elemental analysis (C₂₆H₂₈ClF₂N₃O₃S · H₂O) | | | | | |
|---|---|---|---|---|---|
| Calculated: | C, 56.36%; | H, 5.46%; | N, 7.58%; | Cl, 6.39%; | F, 6.86% |
| Found: | C, 56.50%; | H, 5.1 %; | N, 7.6 %; | Cl, 6.7 %; | F, 6.7 % |

### Example 4: (R,S)-1-(3-Methoxyphenyl)-2-[4-(6,11-dihydrodibenzo[c,f][1,2,5]thiadiazepin-6-ylmethyl)piperidin-1-yl]ethanol S,S-dioxide hydrochloride (hydrochloride of Compound No. 153 shown in Table 1)

### a) 6-[[1-[2-(3-Methoxyphenyl)-2-oxoethyl]-4-piperidinyl]methyl]-6,11-dihydrodibenzo[c,f][1,2,5]thiadiazepine 5,5-dioxide

Using the amine obtained in Example 1(f) (1.4 g, 4.1 mmol) and 2-bromo-3 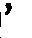 -methoxyacetophenone (940 mg, 4.2 mmol), the title compound was obtained as an oil (yield 90%) according to the method of Example 1(g). Rf=0.5 (methylene chloride/ethyl acetate=1:1).

### b) (R,S)-1-(3-Methoxyphenyl)-2-[4-(6,11-dihydrodibenzo[c,f][1,2,5]thiadiazepine-6-ylmethyl)piperidine-1-yl]ethanol S,S-dioxide hydrochloride

Using the compound obtained in Example 4(a), the title compound was obtained in free form as an oil according to the method of Example 2 (yield: 35%).

The above compound in free form was dissolved in ethyl acetate, and the solution was added with 4N hydrochloric acid/ethyl acetate solution to obtain the title compound as a pale orange solid in 90% yield. Melting point: 160-162°C.

| Elemental analysis value (C₂₇H₃₂ClN₃O₄S · 1/2H₂O) | | | | | |
|---|---|---|---|---|---|
| Calculated: | C, 60.16%; | H, 6.17%; | N, 7.79%; | Cl, 6.58%; | S, 5.95% |
| Found: | C, 60.30%; | H, 6.0 %; | N, 7.7 %; | Cl, 6.1%; | S, 5.7% |

### Example 5: (R,S)-1-(2-Methylphenyl)-2-[4-(6,11-dihydrodibenzo[c,f][1,2,5]thiadiazepin-6-ylmethyl)piperidin-1-yl]ethanol S,S-dioxide hydrochloride (hydrochloride of Compound No. 165 shown in Table 1)

### a) 6-[[1-[2-(2-Methylphenyl)-2-oxoethyl]-4-piperidinyl]methyl]-6,11-dihydrodibenzo[c,f][1,2,5]thiadiazepine 5,5-dioxide

Using the amine obtained in Example 1(f) (1.4 g, 4.1 mmol) and 2-bromo-2 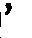 -methylacetophenone (895 mg, 4.2 mmol), the title compound was obtained as an oil according to the method of Example 1(g) (yield: 62%). Rf=0.4 (methylene chloride/ethyl acetate=6:4).

### b) (R,S)-1-(2-Methylphenyl)-2-[4-(6,11-dihydrodibenzo[c,f][1,2,5]thiadiazepin-6-ylmethyl)piperidin-1-yl]ethanol S,S-dioxide hydrochloride

Using the compound obtained in Example 5(a), the title compound was obtained in free form as an oil according to the method of Example 2 (yield: 84%). Rf=0.25 (methylene chloride/ethyl acetate=1:1).

The above compound in free form was dissolved in ethanol, and the solution was added with 4N hydrochloric acid/ethyl acetate solution to obtain the title compound as a white solid in 71% yield. Melting point: 192-194°C.

| Elemental analysis (C₂₇H₃₂ClN₃O₃S · H₂O) | | | | | |
|---|---|---|---|---|---|
| Calculated: | C, 60.95%; | H, 6.44%; | N, 7.90%; | Cl, 6.66%; | S, 6.02% |
| Found: | C, 60.60%; | H, 6.0 %; | N, 7.90%; | Cl, 6.40%; | S, 5.9% |

### Example 6: (R,S)-1-(1-Adamantyl)-2-[4-(11-methyl-6,11-dihydrodibenzo[c,f][1,2,5]thiadiazepin-6-ylmethyl)piperidin-1-yl]ethanol S,S-dioxide hydrochloride (hydrochloride of Compound No. 86 shown in Table 1)

### a) tert-Butyl 4-[6-(11-methyl-6,11-dihydrodibenzo[c,f][1,2,5]thiadiazepine) methyl]piperidine-1-carboxylate S,S-dioxide

A solution of the compound obtained in Example 1(e) (3.55 g, 8 mmol) in dimethylformamide was added with 60% sodium hydride (320 mg, 8 mmol), and the mixture was stirred under nitrogen flow at room temperature for 20 minutes. The solution was then added with methyl iodide (328 µl, 8 mmol), and stirring was continued for ten minutes. The solution was added with water, and extracted with ethyl acetate, and the organic layer was washed with water. The organic layer was dried over sodium sulfate, and the solvent was evaporated under reduced pressure to obtain the title compound as an oil (yield: 96%). Rf=0.3 (ethyl acetate/hexane=3:7).

### b) 4-[6-(11-Methyl-6,11-dihydrodibenzo[c,f][1,2,5]thiadiazepine)methyl]piperidine S,S-dioxide

Deprotection of the compound obtained in Example 6(a) was performed according to the method of Example 1(f) to give the title compound as an oil (yield: 95%).

### c) 6-[[1-[2-(1-Adamantyl)-2-oxoethyl]-4-piperidinyl]methyl]-11-methyl-6,11-dihydrodibenzo[c,f][1,2,5]thiadiazepine 5,5-dioxide

Using the compound obtained in Example 6(b) (1.67 g, 4.7 mmol) and 1-(bromoacetyl)adamantane (1.21 g, 4.7 mmol), the title compound was obtained as an oil according to the method of Example 1(g) (yield: 56%). Rf=0.4 (methylene chloride/ethyl acetate=8:2).

### d) (R,S)-1-(1-Adamantyl)-2-[4-(11-methyl-6,11-dihydrodibenzo[c,f][1,2,5]thiadiazepin-6-ylmethyl)piperidin-1-yl]ethanol S,S-dioxide hydrochloride

Using the compound obtained in Example 6(c), the title compound was obtained in free form as a white solid according to the method of Example 2 (yield: 70%). Melting point: 183-185°C. Rf=0.3 (methylene chloride/ethyl acetate=1:1)

The above compound in free form was dissolved in ethanol, and the solution was added with 4N hydrochloric acid/ethyl acetate solution to obtain the title compound as a white solid (yield: 83%). Melting point: 275-277°C

| Elemental analysis (C₃₁H₄₂ClN₃O₃S) | | | | | |
|---|---|---|---|---|---|
| Calculated: | C, 65.07%; | H, 7.40%; | N, 7.34%; | Cl, 6.20%; | S, 5.60% |
| Found: | C, 64.80%; | H, 7.30%; | N, 7.30%; | Cl, 6.0 %; | S, 5.5 % |

### Example 7: (R,S)-1-(4-Fluorophenyl)-2-[4-(11-methyl-6,11-dihydrodibenzo[c,f][1,2,5]thiadiazepin-6-ylmethyl)piperidin-1-yl]ethanol S,S-dioxide hydrochloride (hydrochloride of Compound No. 337 shown in Table 1)

### a) 6-[[1-[2-(4-Fluorophenyl)-2-oxoethyl]-4-piperidinyl]methyl]-11-methyl-6,11-dihydrodibenzo[c,f][1,2,5]thiadiazepine 5,5-dioxide

Using the compound obtained in Example 6(b) (1.61 g, 4.5 mmol) and 2-chloro-4 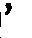 -fluoroacetophenone (777 mg, 4.5 mmol) in the presence of sodium iodide (145 mg, 0.9 mmol), the title compound was obtained as an oil according to the method of Example 1(g) (yield: 73%). Rf=0.5 (methylene chloride/ethyl acetate=8:2).

### b) (R,S)-1-(4-Fluoropheny)-2-[4-(11-methyl-6,11-dihydrodibenzo[c,f][1,2,5]thiadiazepin-6-ylmethyl)piperidin-1-yl]ethanol S,S-dioxide hydrochloride

Using the compound obtained in Example 7(a), the title compound was obtained in free form as a white solid according to the method of Example 2 (yield: 70%). Melting point: 161-163°C. Rf=0.3 (methylene chloride/ethyl acetate=1:1)

The above compound in free form was dissolved in ethanol, and the solution was added with 4N hydrochloric acid/ethyl acetate solution to obtain the title compound as a pale orange solid. Melting point: 164-166°C

| Elemental analysis (C₂₇H₃₁ClFN₃O₃S · 2/3H₂O) | | | | | |
|---|---|---|---|---|---|
| Calculated: | C, 59.60%; | H, 5.99%; | N, 7.72%; | F, 3.49%; | S, 5.90% |
| Found: | C, 59.50%; | H, 5.7 %; | N, 7.7 %; | F, 3.3 %; | S, 6.3% |

### Example 8: (R,S)-1-(1-Adamantyl)-2-[4-(8-chloro-6,11-dihydrodibenzo[c,f][1,2,5]thiadiazepin-6-ylmethyl)piperidin-1-yl]ethanol S,S-dioxide hydrochloride (hydrochloride of Compound No. 489 shown in Table 1)

### a) tert-Butyl 4-[N-(2-acetaminophenylsulfonyl)-N-(2,5-dichlorophenyl)aminomethyl]piperidine-1-carboxylate

Using N-(2,5-dichlorophenyl)-2-acetaminophenylsulfonamide, N-alkylation was performed according to the method of Example 1(d) to obtain the title compound as a brown solid (yield: 52%). Melting point: 125-127°C. Rf=0.6 (ethyl acetate/ hexane=4:6).

### b) tert-Butyl 4-[6-(8-chloro-6,11-dihydrodibenzo[c,f][1,2,5]thiadiazepine)methyl]piperidine-1-carboxylate S,S-dioxide

Using the compound obtained in Example 8(a) (9.6 g, 17.3 mmol), cyclization was performed according to the method of Example 1(e) to obtain the title compound as a pale pink solid (3.5 g, yield: 43%). Melting point: 197-199°C. Rf=0.3 (ethyl acetate/hexane=7:3).

### c) 4-[6-(8-Chloro-6,11-dihydrodibenzo[c,f][1,2,5]thiadiazepine)methyl]piperidine S,S-dioxide

Deprotection of the compound obtained in Example 8(b) was performed according to the method of Example 1(f) to obtain the title compound as a white solid (quantitative). Melting point: 172-174°C.

### d) 6-[[1-[2-(1-Adamantyl)-2-oxoethyl]-4-piperidinyl]methyl]-8-chloro-6,11-dihydrodibenzo[c,f][1,2,5]thiadiazepine 5,5-dioxide

Using the compound which was obtained in Example 8(c) (1.24 g, 3.3 mmol) and 1-(bromoacetyl)adamantane (900 mg, 3.5 mmol), the title compound was obtained according to the method of Example 1(g) as an oil (yield: 60%). Rf=0.5 (methylene chloride/ethyl acetate=1:1).

### e) (R,S)-1-(1-Adamantyl)-2-[4-(8-chloro-6,11-dihydrodibenzo[c,f][1,2,5]thiadiazepin-6-ylmethyl)piperidin-1-yl]ethanol S,S-dioxide hydrochloride

Using the compound obtained in Example 8(d), the title compound was obtained in free form as a white solid according to the method of Example 2 (yield: 81%). Melting point: 266-268°C. Rf=0.2 (methylene chloride/ethyl acetate=1:1)

The above compound in free form was dissolved in ethanol, and the solution was added with 4N hydrochloric acid/ethyl acetate solution to obtain the title compound as a white solid (yield: 79%). Melting point: 295-297°C.

| Elemental analysis (C₃₀H₃₈Cl₂N₃O₃S · 1/2H₂O) | | | | | |
|---|---|---|---|---|---|
| Calculated: | C, 59.99%; | H, 6.55%; | N, 7.02%; | Cl,11.81%; | S, 5.34% |
| Found: | C, 59.60%; | H, 6.8 %; | N, 6.7 %; | Cl, 11.5 %; | S, 5.0 % |

### Example 9: (R,S)-1-(4-Fluorophenyl)-2-[4-(8-chloro-6,11-dihydrodibenzo[c,f][1,2,5]thiadiazepin-6-ylmethyl)piperidin-1-yl]ethanol S,S-dioxide hydrochloride (hydrochloride of Compound No. 333 shown in Table 1)

### a) 6-[[1-[2-(4-Fluorophenyl)-2-oxoethyl]-4-piperidinyl]methyl]-8-chloro-6,11-dihydrodibenzo[c,f][1,2,5]thiadiazepine 5,5-dioxide

Using the compound obtained in Example 8(c) (1.51 g, 4 mmol) and 2-chloro-4 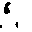 -fluoroacetophenone (690 mg, 4 mmol) in the presence of sodium iodide (145 mg, 0.9 mmol), the title compound was obtained as an oil according to the method of Example 1(g). Rf=0.7 (methylene chloride/ethyl acetate=1:1).

### b) (R,S)-1-(4-Fluorophenyl)-2-[4-(8-chloro-6,11-dihydrodibenzo[c,f][1,2,5]thiadiazepin-6-ylmethyl)piperidin-1-yl]ethanol S,S-dioxide hydrochloride

Using the compound obtained in Example 9(a), the title compound was obtained in free form as a white solid according to the method of Example 2 (yield: 68%). Melting point: 265-267°C. Rf=0.25 (methylene chloride/ethyl acetate=1:1).

The above compound in free form was dissolved in ethanol, and the solution was added with 4N hydrochloric acid/ethyl acetate solution to obtain the title compound as a white solid. Melting point: 190-192°C.

| Elemental analysis (C₃₀H₃₈Cl₂N₃O₃S · 1/2H₂O) | | | | | | |
|---|---|---|---|---|---|---|
| Calculated: | C, 56.52%; | H, 5.11%; | N,7.61%; | Cl,12.83%; | F, 3.44%; | S, 5.80% |
| Found: | C, 55.5 %; | H, 5.1 %; | N,7.3%; | Cl,12.4%; | F, 3.3 %; | S, 5.50% |

### Example 10: (R,S)-1-(1-Adamantyl)-2-[4-(6H-dibenzo[b,f][1,4,5]oxathiazepin-6-yl-methyl)piperidin-1-yl]ethanol S,S-dioxide hemi-fumarate (fumaric acid salt of Compound No. 87 shown in Table 1)

### a) N-(2-Chlorophenylsulfonyl)-2-acetoxyaniline

A solution of 2-aminophenol (2.18 g, 20 mmol) and pyridine (2.42ml, 30 mmol) in ethyl acetate (30 ml) was cooled at 10°C, and added dropwise with a solution of 2-chlorophenylsulfonyl chloride (4.22 g, 20 mmol) in ethyl acetate (15 ml). The reaction mixture was stirred at room temperature for one hour, and then heated under reflux for two hours. After cooling, the reaction mixture was added with water and extracted with ethyl acetate. The organic layer was washed with water, and dried over sodium sulfate, and the solvent was evaporated under reduced pressure. The resulting residue was applied to a silica gel short column (eluent: ethyl acetate/hexane=3:7) to remove tars, and N-(2-chlorophenylsulfonyl)-2-hydroxyaniline was obtained (3.5 g, yield: 61%). Melting point: 97-99°C.

The above compound (4 g, 14 mmol) was dissolved in ethyl acetate (50 ml), and the solution was added dropwise with triethylamine (2.15 ml, 15 mmol) and acetic anhydride (1.4 ml, 15 mmol) with ice cooling. The reaction mixture was stirred at room temperature for one hour, and then added with water and extracted with ethyl acetate. The organic layer was washed with water, and dried over sodium sulfate, and the solvent was evaporated under reduced pressure to obtain the title compound as a white solid (3.8 g, yield: 83%). Melting point: 155-157°C.

### b) tert-Butyl 4-[N-(2-chlorophenylsulfony)-N-(2-hydroxyphenyl)aminomethyl]piperidine-1-carboxylate N-alkylation of the compound obtained in Example 10(a) (6.5 g, 20 mmol) was

performed according to the method of Example 1(d) to obtain tert-butyl 4-[N-(2-chlorophenylsulfonyl)-N-(2-acetoxyphenyl)aminomethyl]piperidine-1-carboxylate as a white solid (4.2 g, yield: 40%). Melting point: 139-140 °C . Rf=0.25 (ethyl acetate/hexane=3:7). The above compound (4.0 g, 7.7 mmol) was dissolved in methanol (30 ml), and the solution was added with aqueous solution (6 ml) of sodium hydroxide (340 mg, 8.5 mmol). After stirring for two hours, the reaction mixture was added with water and 1N hydrochloric acid (10 ml), and extracted with ethyl acetate. The organic layer was washed with water, and dried over sodium sulfate, and the solvent was evaporated under reduced pressure to obtain the title compound as an oil (quantitative). Rf=0.43 (ethyl acetate/hexane=3:7).

### c) tert-Butyl 4-[6-(6H-dibenzo[b,f][1,4,5]oxathiazepine)methyl]piperidine-1-carboxylate S,S-dioxide

Sodium methylate (1.53 g, 28.4 mmol) was added to a solution of the compound obtained in Example 10(b) (10.6 g, 20.26 mmol) in N-methylpyrrolidone (100 ml), and the reaction mixture was stirred at 110°C for six hours. After cooling, the reaction mixture was added with water and extracted with ethyl acetate. The organic layer was washed with water, and dried over sodium sulfate, and the solvent was evaporated under reduced pressure to obtain the title compound as a white solid (9.9 g, yield: 66%). Melting point: 115-117°C. Rf=0.45 (ethyl acetate/hexane=3:7).

### d) 4-[6-(6H-Dibenzo[b,f][1,4,5]oxathiazepine)methyl]piperidine S,S-dioxide

Deprotection of the compound obtained in Example 10(c) was performed according to the method of Example 1(f) to obtain the title compound as an oil (quantitative).

### e) 6-[[1-[2-(1-Adamantyl)-2-oxoethyl]-4-piperidinyl]methyl]-6H-dibenzo[b,f][1,4,5]oxathiazepine 5,5-dioxide

Using the compound obtained in Example 10(d) (1.45 g, 4.2 mmol) and 1-(bromoacetyl)adamantane (1.1 g, 4.2 mmol), the title compound was obtained as an oil according to the method of Example 1(g) (yield: 90%). Rf=0.5 (methylene chloride/ethyl acetate=7:3).

### f) (R,S)-1-(1-Adamantyl)-2-[4-(6H-dibenzo[b,f][1,4,5]oxathiazepin-6-ylmethyl)-piperidin-1-yl]ethanol S,S-dioxide hemi-fumarate

Using the compound obtained in Example 10(e), the title compound was obtained in free form as a white solid according to the method of Example 2 (yield: 73%). Melting point: 208-210°C. Rf=0.25 (methylene chloride/ethyl acetate=7:3).

The above compound in free form was dissolved in ethanol, and the solution was added with a solution of fumaric acid in ethyl acetate to obtain the title compound as a white solid (yield: 54%). Melting point: 211-213°C.

| Elemental analysis (C₃₂H₄₀N₂O₆S · H₂O) | | | | |
|---|---|---|---|---|
| Calculated: | C, 64.20%; | H, 7.07%; | N, 4.68%; | S, 5.52% |
| Found: | C, 64.00%; | H, 7.0 %; | N, 4.80%; | S, 5.30% |

### Example 11: (R,S)-1-(4-Chlorophenyl)-2-[4-(6H-dibenzo[b,f][1,4,5]oxathiazepin-6-ylmethyl)piperidin-1-yl]ethanol S,S-dioxide hydrochloride (hydrochloride of Compound No. 99 shown in Table 1)

### a) 6-[[1-[2-(4-Chlorophenyl)-2-oxoethyl]-4-piperidinyl]methyl]-6H-dibenzo[b,f][1,4,5]oxathiazepine 5,5-dioxide

Using the compound obtained in Example 10(d) (1.4 g, 4.1 mmol) and 2-bromo-4 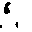 -chloroacetophenone (957 mg, 4.1 mmol), the title compound was obtained as a white solid according to the method of Example 1(g) (yield: 74%). Melting point: 167-169°C. Rf=0.4 (methylene chloride/ethyl acetate=9:1).

### b) (R,S)-1-(4-Chlorophenyl)-2-[4-(6H-dibenzo[b,f][1,4,5]oxathiazepin-6-ylmethyl)piperidin-1-yl]ethanol S,S-dioxide hydrochloride

Using the compound obtained in Example 11(a), the title compound was obtained in free form as a white solid according to the method of Example 2 (yield: 72%). Melting point: 185-187°C. Rf=0.5 (methylene chloride/ethyl acetate=1:1)

The above compound in free form was dissolved in ethanol, and the solution was added with 4N hydrochloric acid/ethyl acetate solution to obtain the title compound as a white solid (yield: 87%). Melting point: 240-242°C.

| Elemental analysis (C₂₆H₂₈Cl₂N₂O₄S) | | | | | |
|---|---|---|---|---|---|
| Calculated: | C, 58.32%; | H, 5.27%; | N, 5.23%; | Cl, 13.24%; | S, 5.99% |
| Found: | C, 58.2 %; | H, 5.5 %; | N, 5.1 %; | Cl,13.2 %; | S, 6.0% |

### Example 12: (R,S)-1-(4-Methoxyphenyl)-2-[4-(6H-dibenzo[b,f][1,4,5]oxathiazepin-6-ylmethyl)piperidin-1-yl]ethanol S,S-dioxide hydrochloride (hydrochloride of Compound No. 103 shown in Table 1)

### a) 6-[[1-[2-(4-Methoxyphenyl)-2-oxoethyl]-4-piperidinyl]methyl]-6H-dibenzo[b,f][1,4,5]oxathiazepine 5,5-dioxide

Using the compound obtained in Example 10(d) (1.4 g, 4.1 mmol) and 2-bromo-4 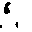 -methoxyacetophenone (939 mg, 4.1 mmol), the title compound was obtained as an oil according to the method of Example 1(g) (yield: 79%). Rf=0.5 (methylene chloride/ethyl acetate=6:4).

### b) (R,S)-1-(4-Methoxyphenyl)-2-[4-(6H-dibenzo[b,f][1,4,5]oxathiazepin-6-ylmethyl)piperidin-1-yl]ethanol S,S-dioxide hydrochloride

Using the compound obtained in Example 12(a), the title compound was obtained in free form as a white solid according to the method of Example 2 (yield: 74%). Melting point: 191-193°C. Rf=0.2 (methylene chloride/ethyl acetate=1:1).

The above compound in free form was dissolved in ethanol, and the solution was added with 4N hydrochloric acid/ethyl acetate solution to obtain the title compound as a white solid (yield: 88%). Melting point: 230-232°C.

| Elemental analysis (C₂₇H₃₁ClN₂O₅S) | | | | | |
|---|---|---|---|---|---|
| Calculated: | C, 61.06%; | H, 5.88%; | N, 5.27%; | Cl, 6.68%; | S, 6.04% |
| Found: | C, 60.9 %; | H, 6.0 %; | N, 5.2 %; | Cl, 6.5 %; | S, 5.9 % |

### Example 13: (R,S)-1-(4-Methylphenyl)-2-[4-(6H-dibenzo[b,f][1,4,5]oxathiazepin-6-ylmethyl)piperidin-1-yl]ethanol S,S-dioxide hydrochloride (hydrochloride of Compound No. 107 shown in Table 1)

### a) 6-[[1-[2-(4-Methylphenyl)-2-oxoethyl]-4-piperidinyl]methyl]-6H-dibenzo[b,f][1,4,5]oxathiazepine 5,5-dioxide

Using the compound obtained in Example 10(d) (1.4 g, 4.1 mmol) and 2-bromo-4 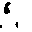 -methylacetophenone (873 mg, 4.1 mmol), the title compound was obtained as a yellow solid according to the method of Example 1(g) (yield: 72%). Melting point: 173-175°C. Rf=0.6 (methylene chloride/ethyl acetate=1:1).

### b) (R,S)-1-(4-Methylphenyl)-2-[4-(6H-dibenzo[b,f][1,4,5]oxathiazepin-6-ylmethyl)piperidin-1-yl]ethanol S,S-dioxide hydrochloride

Using the compound obtained in Example 13(a), the title compound was obtained in free form as a white solid according to the method of Example 2 (yield: 77%). Melting point: 195-197°C. Rf=0.4 (methylene chloride/ethyl acetate=1:1)

The above free compound was dissolved in ethanol, and the solution was added with 4N hydrochloric acid/ethyl acetate solution to obtain the title compound as a white solid (yield: 58%). Melting point: 192-194°C.

| Elemental analysis value (C₂₇H₃₁ClN₂O₄S · 1/2H₂O) | | | | | |
|---|---|---|---|---|---|
| Calculated: | C, 61.88%; | H, 6.15%; | N, 5.34%; | Cl, 6.76%; | S, 6.12% |
| Found: | C, 62.3 %; | H, 6.2 %; | N, 5.3 %; | Cl, 6.8 %; | S, 6.1 % |

### Example 14: (R,S)-1-(1-Adamantyl)-2-[4-(3-chloro-6H-dibenzo[b,f][1,4,5]oxathiazepin-6-ylmethyl)piperidin-1-yl]ethanol S,S-dioxide hydrochloride (hydrochloride of Compound No. 490 shown in Table 1)

### a) N-(2,5-Dichlorophenylsulfonyl)-2-acetoxyaniline

Using 2-aminophenol and 2,5-dichlorophenylsulfonyl chloride, the title compound was obtained as white solid according to the method of Example 10(a) (yield: 41%). Melting point: 100-102°C

### b) tert-Butyl 4-[N-(2,5-dichlorophenylsulfonyl)-N-(2-hydroxyphenyl)aminomethyl]piperidine-1-carboxylate

N-alkylation of the compound obtained in Example 14(a) (5.04 g, 14 mmol) was performed according to the method of Example 1(d) to obtain tert-butyl 4-[N-(2,5-dichlorophenylsulfonyl)-N-(2-acetoxyphenyl)aminomethyl]piperidine-1-carboxylate as a white solid (5.1 g, yield: 65%). Melting point: 140-142 °C. Rf=0.35 (ethyl acetate/hexane=3:7). Deacetylation of this compound was performed according to the method of Example 10(b) to obtain the title compound as a pale orange solid (yield: 97%). Melting point: 159-161°C. Rf=0.45 (ethyl acetate/hexane=4:6).

### c) tert-Butyl 4-[6-(3-chloro-6H-dibenzo[b,f][1,4,5]oxathiazepine)methyl]piperidine-1-carboxylate S,S-dioxide

Using the compound obtained in Example 14(b) (9.74 g, 18.6 mmol), the title compound was obtained as a solid according to the method of Example 10(c) (7.3 g, yield: 80%). Melting point: 173-175°C. Rf=0.4 (ethyl acetate/hexane=3:7).

### d) 4-[6-(3-Chloro-6H-dibenzo[b,f][1,4,5]oxathiazepine)methyl]piperidine S,S-dioxide

Deprotection of the compound obtained in Example 14(c) was performed according to the method of Example 1(f) to obtain the title compound as an oil (quantitative).

### e) 6-[[1-[2-(1-Adamantyl)-2-oxoethyl]-4-piperidinyl]methyl]-3-chloro-6H-dibenzo[b,f][1,4,5]oxathiazepine 5,5-dioxide

Using the compound obtained in Example 14(d) (1.94 g, 5 mmol) and 1-(bromoacetyl)adamantane (1.36 g, 5.3 mmol), the title compound was obtained as an oil according to the method of Example 1(g) (2.4 g, yield: 91%). Rf=0.6 (methylene chloride/ethyl acetate=1:1).

### f) (R,S)-1-(1-Adamantyl)-2-[4-(3-chloro-6H-dibenzo[b,f][1,4,5]oxathiazepin-6-yl-methyl)piperidin-1-yl]ethanol S,S-dioxide hydrochloride

Using the compound obtained in Example 14(e), the title compound was obtained in free form as a white solid according to the method of Example 2 (yield: 77%). Melting point: 168-170°C. Rf=0.4 (methylene chloride/ethyl acetate=1:1).

The above compound in free form was dissolved in ethanol, and the solution was added with 4N hydrochloric acid/ethyl acetate solution to obtain the title compound as a white solid (yield: 84%). Melting point: 175-177°C.

| Elemental analysis (C₃₀H₃₈Cl₂N₂O₄S · 4/5H₂O) | | | | | |
|---|---|---|---|---|---|
| Calculated: | C, 59.26%; | H, 6.58%; | N, 4.60%; | Cl, 11.66%; | S, 5.27% |
| Found: | C, 59.60%; | H, 6.80%; | N, 4.3 %; | Cl, 11.2 %; | S, 5.0 % |

### Example 15: (R,S)-1-Phenyl-2-[4-(3-chloro-6H-dibenzo[b,f][1,4,5]oxathiazepin-6-yl-methyl)piperidin-1-yl]ethanol S,S-dioxide hydrochloride (hydrochloride of Compound No. 491 shown in Table 1)

### a) 3-Chloro-6-[[1-(2-phenyl-2-oxoethyl)-4-piperidinyl]methyl]-6H-dibenzo[b,f][1,4,5]oxathiazepine 5,5-dioxide

Using the compound obtained in Example 14(d) (1.9 g, 5 mmol) and 2-bromoacetophenone (990 mg, 5 mmol), the title compound was obtained as an orange solid according to the method of Example 1(g) (yield: 72.5%). Melting point: 150-152°C.

### b) (R,S)-1-Phenyl-2-[4-(3-chloro-6H-dibenzo[b,f][1,4,5]oxathiazepin-6-ylmethyl)piperidin-1-yl]ethanol S,S-dioxide hydrochloride

Using the compound obtained in Example 15(a), the title compound was obtained in free form as a white solid according to the method of Example 2 (yield: 77%). Melting point: 138-140°C. Rf=0.3 (methylene chloride/ethyl acetate=7:3).

The above compound in free form was dissolved in ethanol, and the solution was added with 4N hydrochloric acid/ethyl acetate solution to obtain the title compound as white solid (yield: 77%). Melting point: 241-243°C.

| Elemental analysis (C₂₆H₂₈Cl₂N₂O₄S) | | | | | |
|---|---|---|---|---|---|
| Calculated: | C, 58.32%; | H, 5.27%; | N, 5.23%; | Cl, 13.24%; | S, 5.99% |
| Found: | C, 58.2 %; | H, 5.2 %; | N, 5.2 %; | Cl, 13.2 %; | S, 5.8 % |

### Example 16 (R,S)-1-(4-Fluorophenyl)-2-[4-(3-chloro-6H-dibenzo[b,f][1,4,5]oxathiazepin-6-ylmethyl)piperidin-1-yl]ethanol S,S-dioxide hydrochloride (hydrochloride of Compound No. 346 shown in Table 1)

### a) 3-Chloro-6-[[1-[2-(4-fluorophenyl)-2-oxoethyl]-4-piperidinyl]methyl]-6H-dibenzo[b,f][1,4,5]oxathiazepine 5,5-dioxide

Using the compound obtained in Example 14(d) (1.13 g, 3 mmol) and 2-chloro-4 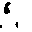 -fluoroacetophenone (518 mg, 3 mmol) in the presence of sodium iodide, the title compound was obtained as a pale yellow solid according to the method of Example 1(g) (yield: 86%). Melting point: 187-189 °C. Rf=0.7 (methylene chloride/ethyl acetate=1:1).

### b) (R,S)-1-(4-Fluorophenyl)-2-[4-(3-chloro-6H-dibenzo[b,f][1,4,5]oxathiazepin-6-yl-methyl)piperidin-1-yl]ethanol S,S-dioxide hydrochloride

Using the compound obtained in Example 16(a), the title compound was obtained in free form as a white solid according to the method of Example 2 (yield: 84%). Melting point: 154-156°C. Rf=0.3 (methylene chloride/ethyl acetate=8:2).

The above compound in free form was dissolved in ethanol, and the solution was added with 4N hydrochloric acid/ethyl acetate solution to obtain the title compound as white solid (yield: 85%). Melting point: 240-242°C.

| Elemental analysis (C₂₆H₂₇Cl₂FN₂O₄S · 1/2H₂O) | | | | | | |
|---|---|---|---|---|---|---|
| Calculated: | C, 55.52%; | H, 5.02%; | N, 4.98%; | Cl, 12.61%; | F, 3.37%; | S, 5.70% |
| Found: | C, 55.60%; | H, 4.9 %; | N, 4.9 %; | Cl,12.7%; | F, 3.2 %; | S, 5.7 % |

### Example 17: (R,S)-1-(2- Naphthyl)-2-[4-(3-chloro-6H-dibenzo[[b,f][1,4,5]oxathiazepin-6-ylmethyl)piperidin-1-yl]ethanol S,S-dioxide hydrochloride (hydrochloride of Compound No. 492 shown in Table 1)

### a) 3-Chloro-6-[[1-[2-(2-naphthyl)-2-oxoethyl]-4-piperidinyl]methyl]-6H-dibenzo[b,f][1,4,5]oxathiazepine 5,5-dioxide

Using the compound obtained in Example 14(d) (980 mg, 2.59 mmol) and 2-bromo-2 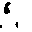 -acetonaphthone (723 mg, 2.9 mmol), the title compound was obtained as a pale yellow solid according to the method of Example 1(g) (yield: 77.6%). Rf=0.25 (hexane/ethyl acetate=2:1).

### b) (R,S)-1-(2-Naphthyl)-2-[4-(3-chloro-6H-dibenzo[b,f][1,4,5]oxathiazepin-6-ylmethyl)piperidin-1-yl]ethanol S,S-dioxide hydrochloride

Using the compound obtained in Example 17(a), the title compound was obtained in free form as a white solid according to the method of Example 2 (yield: 91%). Rf=0.2 (hexane/ethyl acetate=1:1).

The above compound in free form was dissolved in ethyl acetate, and the solution was added with 4N hydrochloric acid/ethyl acetate solution to obtain the title compound as a white solid (yield: 83.7%). Melting point: 240-245°C.

### Example 18: (R,S)-1-(4-Trifluoromethylphenyl)-2-[4-(3-chloro-6H-dibenzo[b,f][1,4,5]oxathiazepin-6-ylmethyl)piperidin-1-yl]ethanol S,S-dioxide hydrochloride (hydrochloride of Compound No. 493 shown in Table 1)

### a) 3-Chloro-6-[[1-[2-(4-trifluoromethylphenyl)-2-oxoethyl]-4-piperidinyl]methyl]-6H-dibenzo[b,f][1,4,5]oxathiazepine 5,5-dioxide

Using the compound obtained in Example 14(d) (1.33 g, 3.51 mmol) and 2-bromo-4 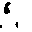 -trifluoromethylacetophenone (1.03 g, 3.86 mmol), the title compound was obtained as a pale yellow solid according to the method of Example 1(g) (yield: 74%). Rf=0.3 (hexane/ethyl acetate=2:1)

### b) (R,S)-1-(4-Trifluoromethylphenyl)-2-[4-(3-chloro-6H-dibenzo[b,f][1,4,5]oxathiazepin-6-ylmethyl)piperidin-1-yl]ethanol S,S-dioxide hydrochloride

Using the compound obtained in Example 18(a), the title compound was obtained in free form as a white solid according to the method of Example 2 (quantitative). Rf=0.3 (hexane/ethyl acetate=1:1)

The above compound in free form was dissolved in ethyl acetate, and the solution was added with 4N hydrochloric acid/ethyl acetate solution to obtain the title compound as white solid (yield: 82%). Melting point: 235-241°C.

### Example 19: (R,S)-1-(4-Trifluoromethoxyphenyl)-2-[4-(3-chloro-6H-dibenzo[b,f][1,4,5]oxathiazepin-6-ylmethyl)piperidin-1-yl]ethanol S,S-dioxide hydrochloride (hydrochloride of Compound No. 494 shown in Table 1)

### a) 3-Chloro-6-[[1-[2-(4-trifluoromethoxyphenyl)-2-oxoethyl]-4-piperidinyl]methyl]-6H-dibenzo[b,f][1,4,5]oxathiazepine 5,5-dioxide

Using the compound obtained in Example 14(d) (1.33 g, 3.51 mmol) and 2-bromo-4 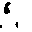 -trifluoromethoxyacetophenone (1.09 g, 3.85 mmol), the title compound was obtained as a yellow solid according to the method of Example 1(g) (yield: 73%). Rf=0.3 (hexane/ethyl acetate=2:1).

### b) (R,S)-1-(4-Trifluoromethylphenyl)-2-[4-(3-chloro-6H-dibenzo[b,f][1,4,5]oxathiazepin-6-ylmethyl)piperidin-1-yl]ethanol S,S-dioxide hydrochloride

Using the compound obtained in Example 19(a), the title compound was obtained in free form as a white solid according to the method of Example 2 (quantitative). Rf=0.3 (hexane/ethyl acetate=1:1)

The above compound in free form was dissolved in ethyl acetate, and the solution was added with 4N hydrochloric acid/ethyl acetate solution to obtain the title compound as a white solid (yield: 84.9%). Melting point: 225-230°C.

### Example 20: (R,S)-1-(1-Adamantyl)-2-[4-(3-chloro-8-methoxy-6H-dibenzo[b,f][1,4,5]oxathiazepin-6-ylmethyl)piperidin-1-yl]ethanol S,S-dioxide hydrochloride (hydrochloride of Compound No. 495 shown in Table 1)

### a) N-(2,5-Dichlorophenylsulfonyl)-2-acetoxy-5-methoxyaniline

Using 2-amino-4-methoxyphenol and 2,5-dichlorophenylsulfonyl chloride, the title compound was obtained as a white solid according to the method of Example 10(a) (yield: 37%). Melting point: 166-168°C.

### b) tert-Butyl 4-[N-(2,5-dichlorophenylsulfonyl)-N-(2-hydroxy-5-methoxyphenyl)aminomethyl]piperidine-1-carboxylate

N-Alkylation of the compound obtained in Example 20(a) (8.3 g, 21 mmol) was performed according to the method of Example 1(d) to obtain tert-butyl 4-[N-(2,5-dichlorophenylsulfonyl)-N-(2-acetoxy-5-methoxyphenyl)aminomethyl]piperidine-1-carboxylate as a white solid (10.2 g, yield: 83%). Melting point: 149-151°C. Rf=0.3 (ethyl acetate/hexane=3:7). Deacetylation of this compound was performed according to the method of Example 10(b) to obtain the title compound as an oil (quantitative). Rf=0.25 (ethyl acetate/hexane=3:7).

### c) tert-Butyl 4-[6-(3-chloro-8-methoxy-6H-dibenzo[b,f][1,4,5]oxathiazepine)methyl]piperidine-1-carboxylate S,S-dioxide

Using the compound obtained in Example 20(b) (9.48 g, 17.4 mmol), the title compound was obtained as a solid according to the method of Example 10(c) (6.7 g, yield: 76%). Melting point: 147-149°C. Rf=0.4 (ethyl acetate/hexane=3:7).

### d) 4-[6-(3-Chloro-8-methoxy-6H-dibenzo[b,f][1,4,5]oxathiazepine)methyl]piperidine S,S-dioxide

Deprotection of the compound obtained in Example 20(c) was performed according to the method of Example 1(f) to obtain the title compound as an oil (quantitative).

### e) 6-[[1-[2-(1-Adamantyl)-2-oxoethyl]-4-piperidinyl]methyl]-3-chloro-8-methoxy-6H-dibenzo[b,f][1,4,5]oxathiazepine 5,5-dioxide

Using the compound obtained in Example 20(d) (1.6 g, 4 mmol) and 1-(bromo acetyl)adamantane (1.03 g, 4 mmol), the title compound was obtained as a yellow solid according to the method of Example 1(g) (yield: 73%). Melting point: 116-118°C. Rf=0.6 (methylene chloride/ethyl acetate=1:1).

### f) (R,S)-1-(1-Adamantyl)-2-[4-(3-chloro-8-methoxy-6H-dibenzo[b,f][1,4,5]oxathiazepin-6-ylmethyl)piperidin-1-yl]ethanol S,S-dioxide hydrochloride

Using the compound obtained in Example 20(e), the title compound was obtained in free form as an oil according to the method of Example 2 (quantitative). Rf=0.2 (methylene chloride/ethyl acetate=1:1).

The above compound in free form was dissolved in ethanol, and the solution was added with 4N hydrochloric acid/ethyl acetate solution to obtain the title compound as a white solid (yield: 69%). Melting point: 254-256°C.

| Elemental analysis (C₃₁H₄₀Cl₂N₂O₅S · 1/2H₂O) | | | | | |
|---|---|---|---|---|---|
| Calculated: | C, 58.86%; | H, 6.53%; | N, 4.43%; | Cl, 11.21%; | S, 5.07% |
| Found: | C, 59.0 %; | H, 6.5 %; | N, 4.4 %; | Cl, 11.2 %; | S, 4.9 % |

### Example 21: (R,S)-1-(4-Fluorophenyl)-2-[4-(3-chloro-8-methoxy-6H-dibenzo[b,f][1,4,5]oxathiazepin-6-ylmethyl)piperidin-1-yl]ethanol S,S-dioxide hydrochloride (hydrochloride of Compound No. 352 shown in Table 1)

### a) 6-[[1-[2-(4-Fluorophenyl)-2-oxoethyl]-4-piperidinyl]methyl]-3-chloro-8-methoxy-6H-dibenzo[b,f][1,4,5]oxathiazepine 5,5-dioxide

Using the compound obtained in Example 20(d) (1.6 g, 4 mmol) and 2-chloro-4 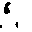 -fluoroacetophenone (690 mg, 4 mmol) in the presence of sodium iodide, the title compound was obtained as an oil according to the method of Example 1(g) (yield: 55%). Rf=0.75 (methylene chloride/ethyl acetate=1:1).

### b) (R,S)-1-(4-Fluorophenyl)-2-[4-(3-chloro-8-methoxy-6H-dibenzo[b,f][1,4,5]oxathiazepin-6-ylmethyl)piperidin-1-yl]ethanol S,S-dioxide hydrochloride

Using the compound obtained in Example 21(a), the title compound was obtained in free form as an oil according to the method of Example 2 (yield: 85%). Rf=0.3 (methylene chloride/ethyl acetate=1:1).

The above compound in free form was dissolved in ethanol, and the solution was added with 4N hydrochloric acid/ethyl acetate solution to obtain the title compound as white solid (yield: 81%). Melting point: 242-244°C.

| Elemental analysis (C₂₇H₂₉Cl₂FN₂O₅S) | | | | | | |
|---|---|---|---|---|---|---|
| Calculated: | C, 55.58%; | H, 5.01%; | N, 4.80%; | Cl, 12.15%; | F, 3.25%; | S, 5.49% |
| Found: | C, 55.30%; | H, 5.0 %; | N, 4.80%; | Cl, 11.9 %; | F, 3.1 %; | S, 5.30% |

### Example 22: (R,S)-1-(4-Fluorophenyl)-2-[4-(8-methoxy-6H-dibenzo[b,f][1,4,5]oxathiazepin-6-ylmethyl)piperidin-1-yl]ethanol S,S-dioxide hydrochloride (hydrochloride of Compound No. 351 shown in Table 1)

### a) tert-Butyl 4-[6-(8-methoxy-6H-dibenzo[b,f][1,4,5]oxathiazepine)methyl]piperidine-1-carboxylate S,S-dioxide

The compound obtained in Example 20(c) (2.54 g, 5 mmol) was dissolved in a mixed solvent of methanol (60 ml) and tetrahydrofuran (20 ml), and the solution was added with 10% palladium-carbon (500 mg) and ammonium formate (1.5 g, 23.8 mmol). After stirring for three hours, palladium-carbon was removed by filtration, and the filtrate was added with water and extracted with ethyl acetate. The organic layer was washed with water, and dried over sodium sulfate, and the solvent was evaporated under reduced pressure to obtain the title compound as an oil (2.2 g, yield: 92.8%). Rf=0.35 (ethyl acetate/hexane=3:7).

### b) 4-[6-(8-Methoxy-6H-dibenzo[b,f][1,4,5]oxathiazepine)methyl]piperidine S,S-dioxide

Deprotection of the compound obtained in Example 22(a) was performed according to the method of Example 1(f) to obtain the title compound as an oil (quantitative).

### c) 6-[[1-[2-(4-Fluorophenyl)-2-oxoethyl]-4-piperidinyl]methyl]-8-methoxy-6H-dibenzo[b,f][1,4,5]oxathiazepine 5,5-dioxide

Using the compound obtained in Example 22(b) (1.9 g, 5 mmol) and 2-chloro-4 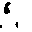 -fluoroacetophenone (863 mg, 5 mmol) in the presence of sodium iodide, the title compound was obtained as an oil according to the method of Example 1(g) (yield: 85%). Rf=0.65 (methylene chloride/ethyl acetate=1:1).

### d) (R,S)-1-(4-Fluorophenyl)-2-[4-(8-methoxy-6H-dibenzo[b,f][1,4,5]oxathiazepin-6-ylmethyl)piperidin-1-yl]ethanol S,S-dioxide hydrochloride

Using the compound obtained in Example 22(c), the title compound was obtained in free form as an oil according to the method of Example 2 (yield: 66%). Rf=0.45 (methylene chloride/methanol=95:5).

The above compound in free form was dissolved in ethanol, and the solution was added with 4N hydrochloric acid/ethyl acetate solution to obtain the title compound as a white solid (yield: 82%). Melting point: 208-210°C.

| Elemental analysis (C₂₇H₃₀ClFN₂O₅S) | | | | | | |
|---|---|---|---|---|---|---|
| Calculated: | C, 59.06%; | H, 5.51%; | N, 5.10%; | Cl,6.46%; | F, 3.46%; | S, 5.84% |
| Found: | C, 58.9 %; | H, 5.5 %; | N, 5.1 %; | Cl,6.2 %; | F, 3.3 %; | S, 5.60% |

### Example 23: (R,S)-1-(1-Adamantyl)-2-[N-methyl-N-[4-(3-chloro-6H-dibenzo[b,f][1,4,5]oxathiazepin-6-yl)butyl]amino]ethanol S,S-dioxide hydrochloride (hydrochloride of Compound No. 499 shown in Table 1)

### a) 4-(N-tert-Butoxycarbonyl-N-methylamino)butyric acid

Using 4-(N-methylamino)butyric acid hydrochloride (23 g, 150 mmol) and di-tert-butyl dicarbonate (32.7 g, 150 mmol), the title compound was obtained as an oil according to the method of Example 1(a) (31 g, yield: 95%).

### b) 4-(N-tert-Butoxycarbonyl-N-methylamino)butanol

Reduction of the compound obtained in Example 23(a) (31 g, 142 mmol) was performed according to the method of Example 1(b) to obtain the title compound as an oil (19.5 g, yield: 64%).

### c) tert-Butyl N-methyl-N-[4-[N-(2,5-dichlorophenylsulfonyl)-N-(2-hydroxyphenyl)]aminobutyl]amine-1-carboxylate

Using the compound obtained in Example 23(b), methanesulfonic acid ester of 4-(N-tert-butoxycarbonyl-N-methylamino)butanol was obtained as an oil according to the method of Example 1(c) (yield: 98%). Using the above compound (11.5 g, 41 mmol) and the compound obtained in Example 14(a) (14.4 g, 40 mmol), N-alkylation was performed according to the method of Example 1(d) to obtain tert-butyl N-methyl-N-[4-[N-(2,5-dichlorophenylsulfonyl)-N-(2-acetoxyphenyl)]aminobutyl]amine-1- carboxylate as an oil. Rf=0.35 (ethyl acetate/hexane=3:7).

Deacetylation of the resulting compound was performed according to the method of Example 10(b) to obtain the title compound as an oil (quantitative). Rf=0.2 (ethyl acetate/hexane=2:8).

### d) tert-Butyl N-methyl-N-[4-(3-chloro-6H-dibenzo[b,f][1,4,5]oxathiazepin-6-yl)butyl]amine-1-carboxylate S,S-dioxide

Using the compound obtained in Example 23(c) (20 g, 40 mmol), the title compound was obtained as an oil according to the method of Example 10(c) (12.2 g, yield: 65%). Rf=0.35 (ethyl acetate/hexane=2:8).

### e) N-Methyl-N-[4-(3-chloro-6H-dibenzo[b,f][1,4,5]oxathiazepin-6-yl)butyl]amine S,S-dioxide

Deprotection of the compound obtained in Example 23(d) was performed according to the method of Example 1(f) to obtain the title compound as an oil (yield: 93%).

### f) 6-[4-[N-[2-(1-Adamantyl)-2-oxoethyl]-N-methyl]aminobutyl]-3-chloro-6H-dibenzo[b,f][1,4,5]oxathiazepine 5,5-dioxide

Using the compound obtained in Example 23(e) (2.56 g, 7 mmol) and 1-(bromoacetyl)adamantane (1.8 g, 7 mmol), the title compound was obtained as an oil according to the method of Example 1(g) (quantitative). Rf=0.5 (methylene chloride/ethyl acetate=1:1).

### g) (R,S)-1-(1-Adamantyl)-2-[N-methyl-N-[4-(3-chloro-6H-dibenzo[b,f][1,4,5]oxathiazepin-6-yl)butyl]amino]ethanol S,S-dioxide hydrochloride

Using the compound obtained in Example 23(f), the title compound was obtained in free form as a white solid according to the method of Example 2 (yield: 80%). Melting point: 99-101°C. Rf=0.5 (methylene chloride/methanol=9:1).

The above compound in free form was dissolved in ethanol, and the solution was added with 4N hydrochloric acid/ethyl acetate solution to obtain the title compound as a white solid (yield: 69%). Melting point: 236-238°C.

| Elemental analysis (C₂₉H₃₈Cl₂N₂O₄S) | | | | | |
|---|---|---|---|---|---|
| Calculated: | C, 59.89%; | H, 6.58%; | N, 4.82%; | Cl, 12.19%; | S, 5.51% |
| Found: | C, 59.5 %; | H, 6.6 %; | N, 4.8 %; | Cl, 11.9 %; | S, 5.3 % |

### Example 24: (R,S)-1-(4-Fluorophenyl)-2-[N-methyl-N-[4-(3-chloro-6H-dibenzo[b,f][1,4,5]oxathiazepin-6-yl)butyl]amino]ethanol S,S-dioxide hydrochloride (hydrochloride of Compound No. 186 shown in Table 1)

### a) 6-[4-[N-[2-(4-Fluorophenyl)-2-oxoethyl]-N-methyl]aminobutyl]-3-chloro-6H-dibenzo[b,f][1,4,5]oxathiazepine 5,5-dioxide

Using the compound obtained in Example 23(e) (3.55 g, 9.7 mmol) and 2-chloro-4 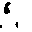 -fluoroacetophenone (1.67 g, 9.7 mmol) in the presence of sodium iodide, the title compound was obtained as an oil according to the method of Example 1(g) (yield: 95%). Rf=0.6 (methylene chloride/ethyl acetate=1:1).

### b) (R,S)-1-(4-Fluorophenyl)-2-[N-methyl-N-[4-(3-chloro-6H-dibenzo][b,f][1,4,5]oxathiazepin-6-yl)butyl]amino]ethanol S,S-dioxide hydrochloride

Using the compound obtained in Example 24(a), the title compound was obtained in free form as an oil according to the method of Example 2 (yield: 77%). Rf=0.3 (methylene chloride/methanol=9:1).

The above compound in free form was dissolved in ethanol, and the solution was added with 4N hydrochloric acid/ethyl acetate solution to obtain the title compound as a white solid (yield: 89%). Melting point: 90-92°C.

| Elemental analysis (C₂₅H₂₇Cl₂FN₂O₄S) | | | | | | |
|---|---|---|---|---|---|---|
| Calculated: | C, 55.46%; | H, 5.03%; | N, 5.17%; | Cl, 13.09%; | F, 3.51%; | S, 5.92% |
| Found: | C, 55.1 %; | H, 5.0%; | N, 5.2%; | Cl, 12.9 %; | F, 3.3 %; | S, 5.7 % |

### Example 25: 3-Chloro-8-fluoro-6-[[1-[2-(4-fluorophenyl)-2-oxoethyl]-4-piperidinyl]methyl]-6H-benzo[b,f][1,4,5]oxathiazepine 5,5-dioxide hydrochloride (hydrochloride of Compound No. 444 shown in Table 1)

### a) N-(2,5-dichlorophenylsulfonyl)-2-acetoxy-5- fluoroaniline

Using 2-amino-4-fluorophenol and 2,5-dichlorophenylsulfonyl chloride, the title compound was obtained as a pale orange solid according to the method of Example 10(a) (yield: 55%). Melting point: 147-149°C.

### b) tert-Butyl 4-[N-(2,5-dichlorophenylsulfonyl)-N-(2-hydroxy-5-fluorophenyl)aminomethyl]piperidine-1-carboxylate

N-Alkylation of the compound obtained in Example 25(a) (20.03 g, 53 mmol) was performed according to the method of Example 1(d) to obtain tert-butyl 4-[N-(2,5-dichlorophenylsulfonyl)-N-(2-acetoxy-5-fluorophenyl)aminomethyl]piperidine-1-carboxylate as a white solid (19.7 g, yield: 65%). Melting point: 145-147°C. Rf=0.3 (ethyl acetate/hexane=3:7). Deacetylation of this compound was performed according to the method of Example 10(b) to obtain the title compound as an oil (quantitative). Rf=0.4 (ethyl acetate/hexane=3:7).

### c) tert-Butyl 4-[6-(3-Chloro-8-fluoro-6H-dibenzo[b,f][1,4,5]oxathiazepine)methyl]piperidine-1-carboxylate S,S-dioxide

Using the compound obtained in Example 25(b) (16.88 mg, 34 mmol), the title compound were obtained as a solid according to the method of Example 10(c) (12.8 g, yield: 75%). Melting point: 152-154°C. Rf=0.6 (ethyl acetate/hexane=3:7).

### d) 4-[6-(3-Chloro-8-fluoro-6H-dibenzo[b,f][1,4,5]oxathiazepine)methyl]piperidine S,S-dioxide

Deprotection of the compound obtained in Example 25(c) was performed according to the method of Example 1(f) to obtain the title compound as an oil (yield: 69%).

### e) 3-Chloro-8-fluoro-6-[[1-[2-(4-fluorophenyl)-2-oxoethyl]-4-piperidinyl]methyl]-6H-dibenzo[b,f][1,4,5]oxathiazepine 5,5-dioxide hydrochloride

Using the compound obtained in Example 25(d) (3.5 g, 8.8 mmol) and 2-chloro-4 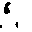 -fluoroacetophenone (1.5 g, 8.8 mmol) in the presence of sodium iodide, the title compound was obtained as a yellow solid according to the method of Example 1(g) (3.3 g, yield: 70%). Melting point: 159-161°C. Rf=0.65 (methylene chloride/ethyl acetate=8:2).

The above compound in free form was dissolved in ethanol, and the solution was added with 4N hydrochloric acid/ethyl acetate solution to obtain the title compound as a white solid (yield: 63%). Melting point: 195-203°C.

### Example 26: (R,S)-1-(4-Fluorophenyl)-2-[4-(3-chloro-8-fluoro-6H-dibenzo[b,f][1,4,5]oxathiazepin-6-ylmethyl)piperidin-1-yl]ethanol S,S-dioxide hydrochloride (hydrochloride of Compound No. 348 shown in Table 1)

Using the compound obtained in Example 25, the title compound was obtained in free form as a white solid according to the method of Example 2 (yield: 81%). Melting point: 134-136°C. Rf=0.6 (methylene chloride/ethyl acetate=1:1).

The above compound in free form was dissolved in ethanol, and added with 4N hydrochloric acid/ethyl acetate solution to obtain the title compound as a white solid (yield: 84%). Melting point: 230-235°C.

### Example 27: 8-Fluoro-6-[[1-[2-(4-fluorophenyl)-2-oxoethyl]-4-piperidinyl]methyl]-6H-dibenzo[b,f][1,4,5]oxathiazepine 5,5-dioxide hydrochloride (hydrochloride of Compound No. 443 shown in Table 1)

### a) tert-Butyl 4-[6-(8-fluoro-6H-dibenzo[b,f][1,4,5]oxathiazepine)methyl]piperidine-1-carboxylate S,S-dioxide

Using the compound obtained in Example 25(c) (7.45 g, 15 mmol), the title compound was obtained as a solid according to the method of Example 22(a) (6.28 g, yield: 90%). Melting point: 184-186°C. Rf=0.6 (ethyl acetate/hexane=3:7).

### b) 4-[6-(8-Fluoro-6H-dibenzo[b,f][1,4,5]oxathiazepine)methyl]piperidine S,S-dioxide

Deprotection of the compound obtained in Example 27(a) was performed according to the method of Example 1(f) to obtain the title compound as an oil (yield: 95%).

### c) 8-Fluoro-6-[[1-[2-(4-fluorophenyl)-2-oxoethyl]-4-piperidinyl]methyl]-6H-dibenzo[b,f][1,4,5]oxathiazepine 5,5-dioxide hydrochloride

Using the compound obtained in Example 27(b) (3.62 g, 10 mmol) and 2-chloro-4 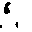 -fluoroacetophenone (1.72 g, 10 mmol) in the presence of sodium iodide, the title compound was obtained as a pale orange solid according to the method of Example 1(g) (yield: 45%). Melting point: 174-177°C. Rf=0.4 (methylene chloride/ethyl acetate=9:1).

The above compound in free form was dissolved in ethanol, and added with 4N hydrochloric acid/ethyl acetate solution to obtain the title compound as a white solid (yield: 56%). Melting point: 208-213°C.

### Example 28: (R,S)-1-(4-Fluorophenyl)-2-[4-(8-fluoro-6H-dibenzo[b,f][1,4,5]oxathiazepin-6-ylmethyl)piperidin-1-yl]ethanol S,S-dioxide hydrochloride (hydrochloride of Compound No. 347 shown in Table 1)

Using the compound obtained in Example 27, the title compound was obtained in free form as a white solid according to the method of Example 2 (yield: 75%). Melting point: 175-177°C. Rf=0.3 (methylene chloride/ethyl acetate=1:1).

The above compound in free form was dissolved in ethanol, and the solution was added with 4N hydrochloric acid/ethyl acetate solution to obtain the title compound as a white solid (yield: 71%). Melting point: 216-220°C.

### Example 29: 8-Chloro-6-[[1-[2-(4-fluorophenyl)-2-oxoethyl]-4-piperidinyl]methyl]-6H-dibenzo[b,f][1,4,5]oxathiazepine 5,5-dioxide hydrochloride (hydrochloride of Compound No. 445 shown in Table 1)

### 2) N-(2-Bromophenylsulfonyl)-2-acetoxy-5-chloroaniline

Using 2-amino-4-chlorophenol and 2-bromophenylsulfonyl chloride, the title compound was obtained as a white solid according to the method of Example 10(a) (yield: 82%). Melting point: 148-150°C.

### b) tert-Butyl 4-[N-(2-bromophenylsulfonyl)-N-(2-dihydroxy-5-chlorophenyl)aminomethyl]piperidine-1-carboxylate

N-Alkylation of the compound obtained in Example 29(a) (24.3 g, 60 mmol) was performed according to the method of Example 1(d) to obtain tert-butyl 4-[N-(2-bromophenylsulfonyl)-N-(2-acetoxy-5-chlorophenyl)aminomethyl]piperidine-1-carboxylate as a white solid (25 g, yield: 70%). Melting point: 155-157°C. Rf=0.2 (ethyl acetate/hexane=2:8). Deacetylation of the above compound was performed according to the method of Example 10(b) to obtain the title compound as an oil (quantitative). Rf=0.2 (ethyl acetate/hexane=2:8).

### c) tert-Butyl 4-[6-(8-chloro-6H-dibenzo[b,f][1,4,5]oxathiazepine)methyl]piperidine-1-carboxylate S,S-dioxide

Using the compound obtained in Example 29(b) (22.9 g, 41 mmol), the title compound was obtained as a white solid according to the method of Example 10(c) (11.3 g, yield: 58%). Melting point: 157-159°C. Rf=0.25 (ethyl acetate/hexane=2:8)

### d) 4-6-(8-Chloro-6H-dibenzo[b,f][1,4,5]oxathiazepine)methyl]piperidine S,S-dioxide

Deprotection of the compound obtained in Example 29(c) was performed according to the method of Example 1(f) to obtain the title compound as an oil (quantitative).

### e) 8-Chloro-6-[[1-[2-(4-fluorophenyl)-2-oxoethyl]-4-piperidinyl]methyl]-6H-dibenzo[b,f][1,4,5]oxathiazepine 5,5-dioxide hydrochloride

Using the compound obtained in Example 29(d) (3.78 g, 10 mmol) and 2-chloro-4 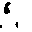 -fluoroacetophenone (1.72 g, 10 mmol) in the presence of sodium iodide, the title compound was obtained as an oil according to the method of Example 1(g) (2.4 g, yield: 47%). Rf=0.5 (methylene chloride/ethyl acetate=8:2).

The above compound in free form was dissolved in ethanol, and the solution was added with 4N hydrochloric acid/ethyl acetate solution to obtain the title compound as a white solid (yield: 56%). Melting point: 209-220°C.

### Example 30: (R,S)-1-(4-Fluorophenyl)-2-[4-(8-chloro-6H-dibenzo[b,f][1,4,5]oxathiazepin-6-ylmethyl)piperidin-1-yl]ethanol S,S-dioxide hydrochloride (hydrochloride of Compound No. 349 shown in Table 1)

Using the compound obtained in Example 29, the title compound was obtained in free form as a white solid according to the method of Example 2 (yield: 96%). Melting point: 157-159°C. Rf=0.5 (methylene chloride/ethyl acetate=1:1).

The above compound in free form was dissolved in ethanol, and the solution was added with 4N hydrochloric acid/ethyl acetate solution to obtain the title compound as a white solid (yield: 66%). Melting point: 206-211°C.

### Test Example: Sigma binding site binding inhibition assay

The sigma 1 selective receptor binding inhibition assay was performed using [³H]-(+)-pentazocine (final concentration: 3 nM, 35 Ci/mmol, New England Nuclear, Dupont de Nemours) as the radioligand according to the method described in literature (D. L. DeHaven-Hudkins, et al., European Journal of Pharmacology-Molecular Pharmacology Section, 1992, 227, 371-378). Crude P2 fraction was prepared from Guinea pig brain whole membrane preparations according to a method described in literature (E. Weber, et al., Proc. Natl. Acad. Sci. USA, 1986, 83, 8784-8788). The membrane fractions (0.4 ml) were allowed to incubate for 150 minutes at 37°C in the presence of various concentrations of the reference compounds (haloperidol: 10⁻¹⁰ to 10⁻⁶ M) or of the test ligand (10⁻¹⁰ to 10⁻⁵ M) and the radioligand in a final volume of 0.5 ml of 50 mM Tris-HCl (pH 7.4).

Assays were terminated by rapid filtration by means of a Brandel cell harvester through Whatman CF/B filters which was soaked in 0.5% polyethyleneimine for one hour prior to use. The filters were washed four times with ice cold incubation buffer. Nonspecific binding was determined using haloperidol (1 µM). The radioactivity on the filters was determined by scintillation spectrometry using Formula 989 (New England Nuclear, Dupont de Nemours) as scintillator liquid and a counter LS 6000 TA (Becmann). Competition curves were analyzed with the curve fitting program ligand (G. A. Mc Pherson, Computer Programs in Biomedicine, 1983, 17, 107). Ki and IC₅₀ values were calculated. Kd value for [³H]-(+)-pentazocine was 3 nM. Values in the table are averages of three experiments.

The sigma 2 selective receptor binding inhibition assay was performed using [³H]-di-o-tolylguanidine (DTG, 1 nM final, 37 Ci/mmol, New England Nuclear, Dupont de Nemours) as the radioligand. Crude P2 membrane fraction was prepared from the livers of male Sprague-Dawley rats according to the method described in literature (X. He, et al., J. Med. Chem. 1993, 36, 566-571). The membrane fractions (0.4 ml) were allowed to inubate for 2 hours at 25°C in the presence of 500 nM of pentazocine and of various concentrations of the reference compound (haloperidol: 10⁻¹⁰ to 10⁻⁶ M) or of the test ligand (10⁻¹⁰ to 10⁻⁵ M) and the radioligand in a final volume of 0.5 ml of 50 mM Tris-HCl (pH 7.4). Assays were terminated by rapid filtration by means of a Brandel cell harvester through Whatman CF/B filters which were soaked in 0.5% polyethyleneimine for one hour prior to use. The filters were washed four times with ice cold incubation buffer. Non specific binding was determined using haloperidol (1 µM). Scintillation counting and curve analysis were determined as previously described. Kd value for [³H]-DTG was 6.9 nM.

| Table 2 | | | |
|---|---|---|---|
| Example No. | Sigma 1 Ki(nM ± SEM) | Sigma 2 Ki(nM ± SEM) | S2/S1 Ratio |
| Haloperidol | 1.6 ± 0.1 | 29 ± 1 | 18 |
| 1 | 150 | 5 | 0.03 |
| 2 | 320 | 4.1 | 0.01 |
| 3 | 280 | 16 | 0.06 |
| 5 | 71 | 13 | 0.2 |
| 6 | 2100 | 2.7 | 0.001 |
| 7 | 540 | 4.0 | 0.007 |
| 8 | 630 | 16 | 0.03 |
| 10 | 200 | 0.76 | 0.004 |
| 11 | 43 | 2.3 | 0.05 |
| 12 | 78 | 7.1 | 0.09 |
| 13 | 89 | 1.6 | 0.02 |
| 14 | 380 | 3.1 | 0.008 |
| 15 | 110 | 1.3 | 0.01 |
| 16 | 85 | 1.1 | 0.01 |
| 17 | 510 | 4.2 | 0.008 |
| 18 | 1100 | 3.2 | 0.003 |
| 19 | 1200 | 8.4 | 0.007 |
| 20 | 3500 | 10 | 0.003 |
| 21 | 420 | 9 | 0.02 |
| 22 | 70 | 3.7 | 0.05 |
| 23 | 1600 | 35 | 0.02 |
| 24 | 140 | 19 | 0.1 |

The above results show that the compounds of the present invention have high affinity for the sigma 2 binding site, and are highly selective sigma 2 ligands. The binding of these compounds to dopaminergic receptors was also measured using rat striata according to the method described by Terai et al. (M. Terai, et al., Eur. J. Pharmacol. 1989, 173, 177). The results are presented in Table 3.

**Table 3**

| Example No. | Dopamine D2 Ki(nM) |
|---|---|
| 1 | >1000 |
| 2 | 450 |
| 3 | 700 |
| 5 | 79 |
| 6 | 170 |
| 7 | 320 |
| 8 | 410 |
| 10 | >1000 |
| 11 | 200 |
| 12 | 280 |
| 13 | 180 |
| 14 | 120 |
| 15 | 180 |
| 16 | 210 |
| 17 | 350 |
| 18 | 350 |
| 19 | 270 |
| 20 | 790 |
| 21 | 820 |
| 22 | 870 |
| 23 | 1100 |
| 24 | 800 |

### Formulation Examples

The pharmaceutical composition of the present invention can be prepared by conventional method in the art using pharmaceutical additives. Typical examples of formulations of the present invention are set out below. However, the pharmaceutical compositions of the present invention are not limited to these examples.

### 1) Tablet

| | |
|---|---|
| Compound of Example 2 | 5-50 mg |
| Calcium phosphate | 20 mg |
| Lactose | 30 mg |
| Talc | 10 mg |
| Magnesium stearate | 5 mg |
| Potato starch | ad 200 mg |

### 2) Suspension

Water is added to 1 to 5 mg of Compound of Example 2, 50 mg of sodium carboxymethylcellulose, 1 mg of sodium benzoate, and 500 mg of sorbitol to prepare an aqueous suspension for oral administration in the total volume of 1 ml.

### 3) Injection

1.5 Percent by weight of Compound of Example 2 as an active ingredient was mixed in a mixture of 10% by volume of propylene glycol and distilled water for injection by stirring, and the resulting solution is sterilized using a membrane filter to prepare a composition for injection.

### 4) Ointment

| | |
|---|---|
| Compound of Example 2 | 5-1, 000 mg |
| Stearyl alcohol | 3 g |
| Lanolin | 5 g |
| White petrolatum | 15 g |
| Water | ad 100 g |

### Industrial Applicability

The compounds of the present invention have high affinity for the sigma 2 binding site, and they have extremely high selectivity for the sigma 2 binding site. Accordingly, the compounds of the present invention are useful as selective sigma 2 ligands for therapeutic and/or preventive treatment of various diseases and symptoms caused by sigma 2 ligands.

## Claims

1. A diarylsultam derivative represented by the following formula:
A-C(X)(Y)-C(R₁)(R₂)-Z
wherein:
Z represents a group represented by formula: 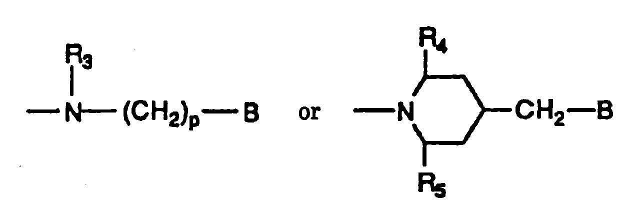 wherein:
R₃ represents an alkyl group, a cycloalkyl group, a hydroxyalkyl group, or an alkenyl group;
p represents an integer of from 3 to 8;
R₄ and R₅ independently represent hydrogen atom or an alkyl group, or R₄ and R₅ together with the intervening atom represent a 5- to 7-membered heterocyclic ring; and
B represents a heteroaryl group of formula: 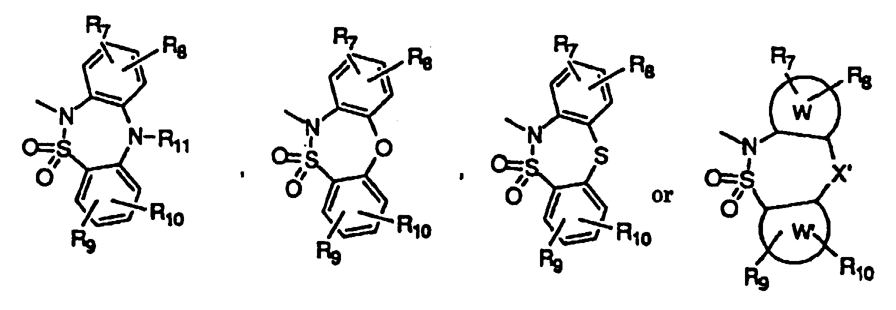 wherein:
R₇, R₈, R₉ and R₁₀ independently represent a substituent selected from the group consisting of hydrogen atom, a halogen atom, nitro group, a cycloalkyl group, an alkenyl group, an alkyl group optionally substituted with 1 to 3 fluorine atoms, hydroxyl group, an alkoxy group optionally substituted with 1 to 3 fluorine atoms, a phenyl group, amino group, an alkylamino group, carbamoyl group, sulfamoyl group, an acyl group, cyano group and an alkynyl group;
X' represents -S-, S(O)-, -S(O)₂-, -O-, or -N(R₁₁)- wherein R₁₁ represents hydrogen atom, an alkyl group or an acyl group; and
W and W' independently represent benzene ring or a 5- to 7-membered heteroaryl group containing one oxygen atom, one sulfur atom, or one or two nitrogen atoms, provided that at least one of W and W' represents the aforementioned heteroaryl group;
X represents a cycloalkyl group, a cycloalkyl-substituted alkyl group, an alkyl-substituted cycloalkyl group, a polycyclic cycloalkyl group, an aryl group, or a heteroaryl group, wherein the aryl group and the heteroaryl group may be substituted with one or more substituents independently selected from the group consisting of a halogen atom, nitro group, a cycloalkyl group, an alkenyl group, an alkyl group optionally substituted with 1 to 3 fluorine atoms, hydroxyl group, an alkoxy group optionally substituted with 1 to 3 fluorine atoms, phenyl group, amino group, an alkylamino group, carboxyl group, carbamoyl group, sulfamoyl group, an acyl group, cyano group, and an alkynyl group;
Y represents hydrogen atom, an alkyl group, an alkenyl group, an alkynyl group, a cycloalkyl group, a cycloalkyl-substituted alkyl group, adamantyl group, an aryl group, an aryl-substituted alkyl group, a heteroaryl group or a heteroaryl-substituted alkyl group, wherein the aryl group and the heteroaryl group may be substituted with one or more substituents independently selected from the group consisting of a halogen atom, nitro group, a cycloalkyl group, an alkenyl group, an alkyl group optionally substituted with 1 to 3 fluorine atoms, hydroxyl group, an alkoxy group optionally substituted with 1 to 3 fluorine atoms, phenyl group, amino group, an alkylamino group, carbamoyl group, sulfamoyl group, an acyl group, cyano group and an alkynyl group;
A is a group represented by -O-R₆ wherein R₆ represents hydrogen atom, an alkyl group, a cycloalkyl group, a cycloalkyl-substituted alkyl group, an alkenyl group, an aryl group, an aryl-substituted alkyl group, a hydroxyalkyl group, an acyl group, or benzoyl group;
Y and A may combine to form oxo group or hydroxylimino group; and
R₁ and R₂, which may be the same or different, independently represent hydrogen atom, an alkyl group, a cycloalkyl group, a hydroxyalkyl group or an alkenyl group,
and a salt thereof, and a hydrate thereof and a solvates thereof

2. The compound according to claim 1 or a salt thereof, or a hydrate thereof or a solvate thereof,
wherein:
R₃ is a substituent selected from the group consisting of an alkyl group having 1 to 3 carbon atoms, a cycloalkyl group having 3 to 6 carbon atoms, a hydroxyalkyl group having 2 or 3 carbon atoms and an alkenyl group having 3 tp 6 carbon atoms;
R₇, R₈, R₉ and R₁₀ are a substituent independently selected from the group consisting of hydrogen atom, a halogen atom, nitro group, an alkyl group having 1 to 6 carbon atoms optionally substituted with 1 to 3 fluorine atoms, hydroxyl group, an alkoxy group having 1 to 6 carbon atoms optionally substituted with 1 to 3 fluorine atoms, phenyl group, amino group, an alkylamino group having 1 to 6 carbon atoms, carbamoyl group, sulfamoyl group, an acyl group having 2 to 6 carbon atoms and cyano group;
R₁₁ is a substituent selected from the group consisting of hydrogen atom, an alkyl group having 1 to 6 carbon atoms and an acyl group having 2 to 6 carbon atoms;
X represents a substituent selected from the group consisting of a cycloalkyl group having 3-6 carbon atoms, an alkyl group having 1-3 carbon atoms substituted with a cycloalkyl group having 3-6 carbon atoms, a cycloalkyl group having 3 to 6 carbon atoms substituted with an alkyl group having 1 to 3 carbon atoms, a polycycloalkyl group having 5 to 12 carbon atoms, an aryl group and a heteroaryl group, wherein the aryl group and the heteroaryl group may be substituted with 1 to 3 substituents independently selected from the group consisting of a halogen atom, nitro group, a cycloalkyl group having 3 to 6 carbon atoms, an alkenyl group having 3 to 6 carbon atoms, an alkyl group having 1 to 6 carbon atoms optionally substituted with 1 to 3 fluorine atoms, hydroxyl group, an alkoxy group having 1 to 6 carbon atoms optionally substituted with 1 to 3 fluorine atoms, phenyl group, amino group, an alkylamino group having 1-6 carbon atoms, carbamoyl group, sulfamoyl group, an acyl group having 2 to 6 carbon atoms, cyano group and an alkynyl group having 3 to 6 carbon atoms;
Y is a substituent selected from the group consisting of hydrogen atom, an alkyl group having 1 to 6 carbon atoms, an alkenyl group having 3 to 6 carbon atoms, an alkynyl group having 3 to 6 carbon atoms, a cycloalkyl group having 3 to 6 carbon atoms, an alkyl group having 1 to 3 carbon atoms substituted with a cycloalkyl group having 3 to 6 carbon atoms, and adamantyl group, or a substituent selected from the group consisting of phenyl group, naphthyl group, and an alkyl group having 1 to 3 carbon atoms substituted with an aryl group;
R₆ is a substituent selected from the group consisting of hydrogen atom, an alkyl group having 1 to 6 carbon atoms, a cycloalkyl group having 3 to 6 carbon atoms, an alkyl group having 1 to 3 carbon atoms substituted with a cycloalkyl group having 3 to 6 carbon atoms, an alkenyl group having 3 to 6 carbon atoms, phenyl group, an alkyl group having 1 to 3 carbon atoms substituted with phenyl group, a hydroxyalkyl group having 2 to 6 carbon atoms, an acyl group having 2 to 6 carbon atoms and benzoyl group;
Y and A may combine to form oxo or hydroxylimino group; and
R₁ and R₂, which may be the same or different, represent a substituent selected from the group consisting of hydrogen atom, an alkyl group having 1 to 3 carbon atoms, a cycloalkyl group having 3 to 6 carbon atoms, a hydroxyalkyl group having 2 or 3 carbon atoms, and an alkenyl group having 3 to 6 carbon atoms.

3. The compound according to claim 1 or a salt thereof, or a hydrate thereof or a solvate thereof,
wherein:
R₃ is an alkyl group having 1 to 3 carbon atoms,
p is an integer of from 3 to 6
R₄ and R₅ are hydrogen atoms;
X' is -S-, -O-, or -N(R₁₁)- wherein R₁₁ is hydrogen atom, an alkyl group having 1-6 carbon atoms, or an acyl group having 2 to 6 carbon atoms;
X represents a substituent selected from the group consisting of a cycloalkyl group having 3 to 6 carbon atoms, an alkyl group having 1 to 3 carbon atoms substituted with a cycloalkyl group having 3 to 6 carbon atoms, a cycloalkyl group having 3 to 6 carbon atoms substituted with an alkyl group having 1 to 3 carbon atoms, and adamantyl group;
Y is hydrogen atom or an alkyl group having 1 to 6 carbon atoms;
R₆ is hydrogen atom, an alkyl group having 1 to 6 carbon atoms, or an acyl group having 2 to 6 carbon atoms;
Y and A may combine to form oxo or hydroxylimino group; and
R₁ and R₂, which may be the same or different, represent hydrogen atom, or an alkyl group having 1 to 3 carbon atoms.

4. The compound according to claim 1 or a salt thereof, or a hydrate thereof or a solvate thereof,
wherein:
R₃ is an alkyl group having 1 to 3 carbon atoms,
p is an integer of from 3 to 6
R₄ and R₅ are hydrogen atoms;
X' is -S-, -O-, or -N(R₁₁)- wherein R₁₁ is hydrogen atom, an alkyl group having 1 to 6 carbon atoms, or an acyl group having 2 to 6 carbon atoms;
X is an aryl group or a heteroaryl group, wherein the aryl group and the heteroaryl group may be substituted with 1 to 3 substituents independently selected from the group consisting of a halogen atom, nitro group, a cycloalkyl group having 3 to 6 carbon atoms, an alkenyl group having 3 to 6 carbon atoms, an alkyl group having 1 to 6 carbon atoms optionally substituted with 1 to 3 fluorine atoms, hydroxyl group, an alkoxy group having 1 to 6 carbon atoms optionally substituted with 1 to 3 fluorine atoms, phenyl group, amino group, an alkylamino group having 1 to 6 carbon atoms, carbamoyl group, sulfamoyl group, an acyl group having 2 to 6 carbon atoms, cyano group and an alkynyl group having 3 to 6 carbon atoms;
Y is hydrogen atom or an alkyl group having 1 to 6 carbon atoms;
R₆ is hydrogen atom, an alkyl group having 1 to 6 carbon atoms, or an acyl group having 2 to 6 carbon atoms;
Y and A may combine to form oxo or hydroxylimino group; and
R₁ and R₂, which may be the same or different, represent hydrogen atom, or an alkyl group having 1-3 carbon atoms.

5. The compounds according to claim 1 or a salt thereof, or a hydrate thereof or a solvate thereof,
wherein:
both of R₁ and R₂ are hydrogen atoms, and R₃ is methyl group;
R₁, R₂, R₄, and R₅ are hydrogen atoms;
p is 4;
A is hydroxy group, methoxy group or acetoxy group;
Y is hydrogen atom or methyl group;
A together with Y represent oxo group or hydroxyimino group; and
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group.

6. The compound according to claim 1 or a salt thereof, or a hydrate thereof or a solvate thereof,
wherein:
B is represented by the following formula. 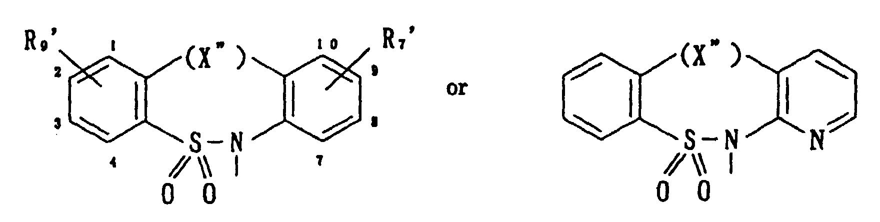
wherein X'' represents -O-, -S-, -NH-, -N(CH₃)- or -N(COCH₃)-; R₇' represents hydrogen atom, 8-F, 9-F, 8-Cl or 8-OCH₃; and R₉' represents hydrogen atom or 3-Cl.

7. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A and Y may form oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

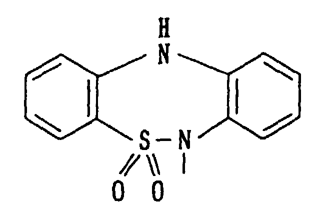

8. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A and Y may form oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

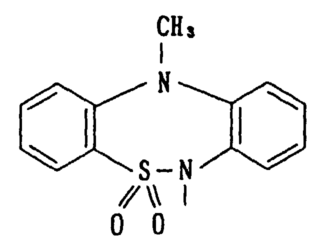

9. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

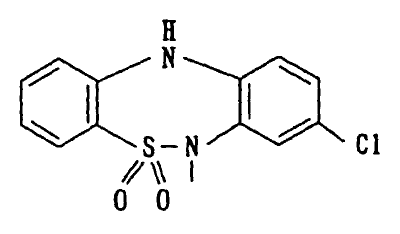

10. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

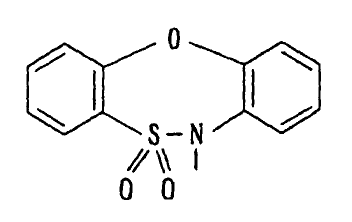

11. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

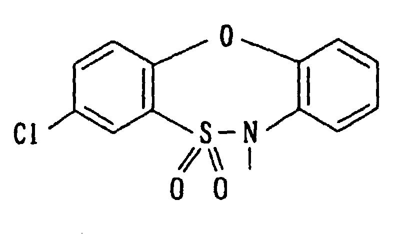

12. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

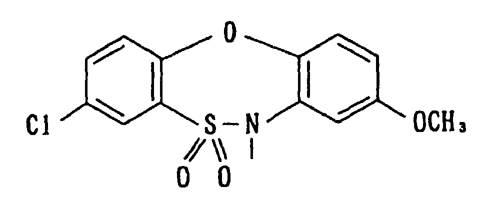

13. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

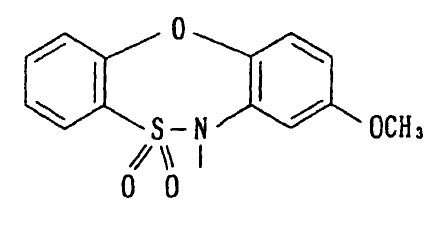

14. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

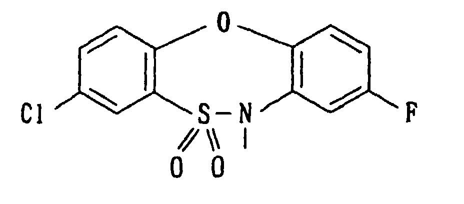

15. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

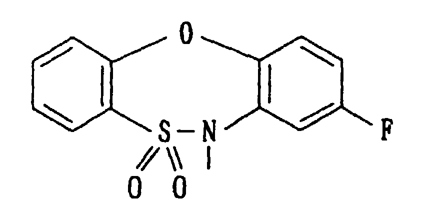

16. The compound according to claim 1 or a salt thereof, or a hydrate thereof or a solvate thereof,
wherein:
both of R₁ and R₂ are hydrogen atoms, and R₃ is methyl group;
R₁, R₂, R₄ and R₅ are hydrogen atoms;
p is 4;
A is hydroxy group;
Y is hydrogen atom;
A together with Y represent oxo group;
X is cyclohexyl group, adamantyl group, phenyl group, 2-naphthyl group, 2,4-difluorophenyl group, 2-methylphenyl group, 4-methylphenyl group, 4-fluorophenyl group, 4-trifluoromethylphenyl group, 4-trifluoromethoxyphenyl group, 4-chlorophenyl group, 3-methoxyphenyl group or 4-methoxyphenyl group; and
B is represented by the following formula.

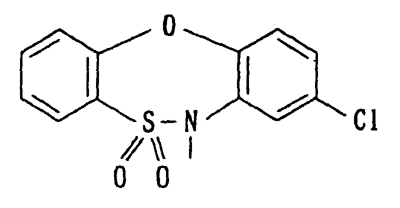

17. A medicament comprising a compound according to any one of claims 1 to 16 or a pharmacologically acceptable salt thereof, or a hydrate thereof and/or a solvate thereof.

18. The medicament according to claim 17 which is used for therapeutic and/or preventive treatment of a disease and/or symptom of a mammal including human affected or facilitated by neuromodulatory effect of a sigma 2 ligand.

19. The medicament according to claim 18 wherein the disease and/or symptom is selected from the group consisting of a central nervous system disease, a gastrointestinal disease, and a cardiovascular system disease.

20. A pharmaceutical composition comprising a compound of any one of claims 1 to 16, a pharmacologically acceptable salt thereof, a hydrate thereof and/or a solvate thereof.

21. A selective sigma 2 ligand comprising a compound of any one of claims 1 to 16, a pharmacologically acceptable salt thereof, a hydrate thereof and/or a solvate thereof.

22. Use of a compound of any one of claims 1 to 16, a salt thereof, a hydrate thereof and/or a solvate thereof for the manufacture of the pharmaceutical composition according to claim 20.

23. A method for therapeutic and/or preventive treatment of a disease and/or symptom affected or facilitated by neuromodulatory effect of a sigma 2 ligand which comprises the step of administering to a patient a therapeutically and/or preventively effective amount of a compound of any one of claims 1 to 16, a pharmacologically acceptable salt thereof, a hydrate thereof and/or a solvate thereof.
